# EUROPEAN PATENT SPECIFICATION

(11) **EP 4 188 496 B1**
(45) Date of publication and mention of the grant of the patent: **28.01.2026**
(21) Application number: 21851064.2
(22) Date of filing: 27.07.2021
(51) Int. Cl.: A61M 16/06, A61M 16/00, A61M 16/20, A62B 7/04

(54) **AUTOMATIC SYSTEM FOR THE CONSERVATION OF OXYGEN AND OTHER SUBSTANCES**
AUTOMATISCHES SYSTEM ZUR KONSERVIERUNG VON SAUERSTOFF UND ANDEREN SUBSTANZEN
SYSTÈME AUTOMATIQUE DE CONSERVATION D'OXYGÈNE ET D'AUTRES SUBSTANCES

(30) Priority: 27.07.2020 US 202063056944 P; 12.10.2020 US 202017068718
(43) Date of publication of application: 07.06.2023
(73) Proprietor: Oxfo Corporation, Chestnut Hill, MA 02467 (US)
(72) Inventor: BAZOBERRY, Carlos, Fernando, Chestnut Hill, MA 02467 (US); YOUNG, Brent, H., Boston, MA 02116 (US)
(74) Representative: Visser-Luirink, Gesina
(86) International application number: PCT/US2021/043333
(87) International publication number: WO 2022/026479

(56) References cited:
- EP-A1- 3 701 992
- WO-A1-2009/026562
- US-A- 3 831 595
- US-A1- 2016 193 438
- US-B1- 6 364 161
- US-B1- 6 484 721
- US-B2- 10 201 674
- US-B2- 7 370 651
- US-B2- 7 588 032
- RITCHIE G ET AL: "CLOSED-LOOP CONTROL OF OXYGEN DELIVERY DURING AEROMEDICAL EVACUATION OF PATIENTS", PROCEEDINGS OF THE SOUTHEAST CONFERENCE (SOUTHEASTCON). BIRMINGHAM, ALABAMA, APR. 12 - 15, 1992; [PROCEEDINGS OF THE SOUTHEAST CONFERENCE (SOUTHEASTCON)], NEW YORK, IEEE, US, vol. 2, 12 April 1992 (1992-04-12), pages 763 - 767, XP000339538, ISBN: 978-0-7803-0494-9, DOI: 10.1109/SECON.1992.202432

## Description

### CROSS-REFERENCE TO RELATED APPLICATIONS

This application claims priority to U.S. Provisional Application No. 63/056,944, filed 07/27/2020, and to U.S. Patent Application No. 17/068,718, filed 10/12/2020.

### TECHNICAL FIELD

The present invention relates generally to the delivery of gases from a source to a recipient. More particularly, disclosed herein are a system and method (the method not being separately claimed) for conserving oxygen and other gases and substances when delivering them from a donor reservoir to a recipient by volumetric displacement at ambient pressure with automatic refilling of the donor reservoir from a source. In embodiments of the system and method, oxygen and other gases and substances are transferred from a donor reservoir to a recipient on demand by the recipient via a pressure difference between them with automatic refilling of the donor reservoir with gas at ambient pressure.

### BACKGROUND OF THE INVENTION

The supply of oxygen can be a critical need for hospital patients and others. Meanwhile, in developing countries and during times of increased demand in all places, shortages of oxygen and excessive costs can place extreme limits on availability and can jeopardize the health and safety of patients in need. For instance, during the COVID-19 pandemic that is ongoing during the writing of this document, the demand for oxygen has left hospitals and other caregiving institutions in dire need of the life-saving gas. One headline from the AP News network on June 24, 2020 warned, "Scarce Medical Oxygen Worldwide Leaves Many Gasping for Life." One day later, Reuters observed, "WHO Warns of Oxygen Shortage as COVID Cases Set to Top 10 Mln" with the World Health Organization estimating based on there being approximately one million new coronavirus cases worldwide per week that the world will need 620,000 cubic meters of oxygen per day, which roughly equals 88,000 large cylinders, for COVID-19 patients alone.

One way that supplemental oxygen is supplied to patients is via a fluidic connection, typically extension tubing, between a pressurized source of oxygen, such as an oxygen cylinder or tank, and the patient. The pressurized source supplies a constant flow of oxygen to the patient as it travels from the tank continuously through the connector tubing, regardless of whether the patient is breathing in or out. As a result, even while the patient exhales and thus cannot intake oxygen, the oxygen constantly flows and is wasted. Indeed, half or even more of the constantly supplied oxygen in this continuous pressure feed method is wasted and expelled to the atmosphere.

It is thus apparent that conserving oxygen resources with one patient may well save the life of another. Moreover, conserving oxygen will not only reduce the overall need per patient but will also tend to reduce the cost of oxygen per unit. Meanwhile, each patient requires a sufficient supply of oxygen. The challenge is thus how to provide oxygen in sufficient supply on demand while minimizing waste.

To comprehend a solution to that challenge, one must understand what drives the flow of air containing oxygen into the lungs, how flow is normally initiated and maintained between the patient and ambient air, and how alveolar pressure varies while pleural pressure decreases throughout inspiration. Air, like other fluids, moves from a region of higher pressure to a region of lower pressure. The flow of air into the lungs requires the establishment of a pressure gradient between the atmosphere and the alveoli. This driving pressure gradient is accomplished by the contraction of the inspiratory muscles. Contraction of the inspiratory muscles expands the chest wall, lowering the pressures in the thoracic cavity so that intra-pleural and alveolar pressure decrease according to Boyle's law. Muscle contraction results in a change in thoracic volume, leading to a change in alveolar pressure, which in turn provides the driving pressure for air flow into the lungs.

Normally, the lungs absorb oxygen from the air during breathing. However, certain conditions can prevent a person from getting enough oxygen. As a result, oxygen therapy with oxygen delivery equipment is required. Patients can receive oxygen therapy from a source of oxygen through tubes resting in their nose, through a facemask, or through a tube placed in their trachea or windpipe. Oxygen treatment increases the amount of oxygen the lungs receive and deliver to the blood. Oxygen therapy may be prescribed for a patient when the patient has a condition that causes the patient's blood oxygen levels to be too low. Low blood oxygen may make patients feel short of breath, tired, or confused and can damage the patient's body. Oxygen long-term basis. Often, the source of oxygen is a tank of compressed oxygen gas or liquid.

Oxygen tanks must be produced, transported, stored, and refilled on a continual basis. In exigent circumstances, such as during an epidemic or a pandemic involving respiratory distress, need can dangerously outpace supply. Moreover, in remote and economically challenged locations, providing ample replenishment of oxygen supplies can be highly costly, even catastrophically impossible. Meanwhile, with life-saving oxygen in preciously short supply and with oxygen constantly passed through tubing to the patient, at least half of the supplied oxygen is simply exhausted into the atmosphere, including during exhalation during which the entirety of the supplied oxygen is wasted. *The non pre-published* EP 3701992 *describes a gas delivery device with deformable bag and differential pressure sensors.* Exemplary systems for the conservation of oxygen supplied to a patient are known from US3831595A and WO2009/026562A1.

In view of the foregoing, the present inventors appreciated the critical need for a system and method capable of providing a ready supply of oxygen to a patient while minimizing or eliminating wasted oxygen thereby to minimize the needs of individual patients, to maximize the effective supply of oxygen, and to enable better patient care and optimal health outcomes in a cost-efficient manner even in times of public health crises.

### SUMMARY DISCLOSURE OF THE INVENTION

Knowing the critical need for ample supplies of oxygen to patients, a primary object of the present invention is to save lives. The invention is defined in the appended independent claim 1. Preferred embodiments of the invention are matter of the dependent claims.

The present inventors further set forth with a basic object of providing a system for supplying oxygen and other flowable substances to patients and other recipients in need that reduces wasted oxygen thereby to maximize the effective use of available oxygen supplies.

A further object of embodiments of the invention is to provide a system for supplying oxygen and other flowable substances to recipients that enable an ample supply of oxygen on-demand while minimizing or eliminating inefficient oxygen losses.

Another object of manifestations of the invention is to provide a system for supplying oxygen and other flowable substances to recipients that maximize efficiencies in usage and minimize supply costs.

These and further objects, advantages, and details of the present invention will become obvious not only to one who reviews the present specification and drawings but also to those who have an opportunity to observe the systems and methods disclosed herein in operation. However, it will be appreciated that, although the accomplishment of plural of the foregoing objects in a single embodiment of the invention may be possible and indeed preferred, not all objects in a single embodiment of the invention may be possible and indeed preferred, not all embodiments will seek or need to accomplish each and every potential advantage and function. Nonetheless, all such embodiments should be considered within the scope of the present invention.

One will appreciate that the foregoing discussion broadly outlines the more important goals and features of the invention to enable a better understanding of the detailed description that follows and to instill a better appreciation of the inventors' contribution to the art. Before any particular embodiment or aspect thereof is explained in detail, it must be made clear that the following details of construction and illustrations of inventive concepts are mere examples of the many possible manifestations of the invention.

In carrying forth one or more of the foregoing objects, an embodiment of the present invention can be characterized as a system for the conservation of oxygen supplied to a patient. The system has an expandable and compressible donor reservoir that has an outer wall, an inner volume for retaining a volume of oxygen, and at least one orifice for allowing a passage of oxygen into and out of the inner volume. The donor reservoir comprises a shell of flexible foil. A supply conduit is adapted to receive oxygen from a source of oxygen. The supply conduit has a first end for supplying oxygen to the donor reservoir and a second end for being fluidically connected to the source of oxygen, and an ambient pressure conduit is adapted to supply oxygen along a fluid path from the donor reservoir to a recipient. The ambient pressure conduit has a first end in fluidic communication with the donor reservoir, such as through a connector, for receiving oxygen from the donor reservoir and a second end for being fluidically connected to the recipient. An inflation detection system is operable to detect a first condition wherein the donor reservoir is inflated with oxygen to a predetermined state of inflation and a second condition wherein the donor reservoir is below the predetermined state of inflation. Finally, a valve system is disposed between the source of oxygen and the donor reservoir. The valve system is operative in a closed condition to prevent oxygen from flowing from the source of oxygen and into the donor reservoir when the donor reservoir is in the first condition, and the valve system is operative in an open condition to permit oxygen to flow from the source of oxygen and into the donor reservoir when the donor reservoir is in the second condition. Under this construction, oxygen can be supplied to a patient, such as through a patient breathing mask as the recipient, from the donor reservoir, and the donor reservoir can be automatically replenished to the predetermined state of inflation.

In practices of the system, the valve system and the inflation detection system are operative to maintain the volume of oxygen in the donor reservoir substantially at ambient pressure. For instance, the donor reservoir can be considered to have a fully inflated condition, and the inflation detection system can be operative to detect when the donor reservoir is inflated to within a predetermined range of the fully inflated condition. The inflation detection system can then detect the first condition when the donor reservoir is inflated to within the predetermined range of the fully inflated condition, and the inflation detection system can detect the second condition when the donor reservoir is inflated below the predetermined range of the fully inflated condition.

**In** certain embodiments, the inflation detection system comprises an electro-mechanical system. For instance, the inflation detection system can comprise a switch disposed to be moved by the outer wall of the donor reservoir when the donor reservoir is inflated with oxygen to the predetermined state of inflation. The switch can be biased, such as by gravity, by a resiliently compressible member, or by any other effective method, toward the donor reservoir. The switch can be considered to have an activated state wherein the switch is disposed at or beyond an inward position with respect to the inner volume of the donor reservoir and a deactivated state when the switch is moved outwardly by the outer wall of the donor reservoir when the volume of oxygen in the donor reservoir reaches the predetermined state of inflation. The valve system is operative to prevent oxygen from flowing from the source of oxygen and into the donor reservoir when the switch is in the deactivated state, and the valve system is operative to permit oxygen to flow from the source of oxygen and into the donor reservoir when the switch is in the activated state.

**In** particular manifestations of the system, the switch comprises a float switch. For example, the float switch can have a contact structure with a collar that is extendable and retractable relative to a central column. The collar can then retain a magnet, and the central column can then retain electrical contacts that are brought into electrical contact by a proximity of the magnet when the switch is in the activated state.

According to practices of the system, the valve system can take the form of a solenoid valve that is in electrical communication with the inflation detection system. The solenoid valve can be induced by the inflation detection system to a closed condition to prevent the flow of oxygen from the source of oxygen to the donor reservoir when the donor reservoir is in the first condition, and the solenoid valve can be induced by the inflation detection system to an open condition to permit the flow of oxygen from the source of oxygen to the donor reservoir when the donor reservoir is in the second condition.

A recipient delivery device, such as a patient breathing mask or another recipient delivery device, can be coupled to the second end of the ambient pressure conduit. Further, in certain embodiments, the donor reservoir can be disposed within a housing, which could comprise a main housing of the system, a sub-housing within a main housing, or some other type of housing. In other practices, the donor reservoir can be disposed without a housing. Where a housing is provided, the inflation detection system can comprise an electro-mechanical system with a switch supported by the housing and disposed to be moved by the outer wall of the donor reservoir when the donor reservoir is inflated with oxygen to the predetermined state of inflation. Even more particularly, the housing can be transparent such that the state of inflation of the donor reservoir can be visually perceived.

Embodiments of the system can further incorporate a one-way inspiratory valve disposed along the fluid path from the donor reservoir to the recipient. The one-way inspiratory valve can be operative to enable oxygen to flow from the donor reservoir, through the ambient pressure conduit, and to the recipient but to prevent reverse flow of oxygen.

In alternative practices of the invention, the inflation detection system comprises a contactless detection system. For instance, the inflation detection system can take the form of an optical detection system.

While the present invention is largely described as being employed to supply oxygen to human or other living patients in a manner that conserves oxygen supply, it will be understood that the invention is not limited to retaining and dispensing oxygen. Indeed, other gases and mixtures of gases and other fluids are possible within the scope of the invention. To that extent, embodiments of the disclosed technology can be more broadly characterized as a system for providing a supply of gas. Moreover, the gas need not necessarily be supplied to a patient. Other recipients are contemplated and within the scope of the disclosed technology.

One will appreciate that the foregoing discussion broadly outlines the more important goals and features of the invention to enable a better understanding of the detailed description that follows and to instill a better appreciation of the inventors' contribution to the art. Before any particular embodiment or aspect thereof is explained in detail, it must be made clear that the following details of construction and illustrations of inventive concepts are mere examples of the many possible manifestations of the invention.

### BRIEF DESCRIPTION OF THE DRAWINGS

In the accompanying drawing figures:
FIG. 1 is a schematic view of an automatic system for the conservation of gas according to the present invention;
FIG. 2 is a schematic view depicting a series of respiratory cycles employing the automatic system for the conservation of gas as disclosed herein;
FIG. 3 is a top plan view of an alternative embodiment of the automatic system for the conservation of gas;
FIG. 4 is a view in front elevation of the automatic system for the conservation of gas of FIG. 3;
FIG. 5 is a lateral perspective view of the automatic system for the conservation of gas of FIG. 3;
FIG. 6 is an upper perspective view of an inflation detection system for the automatic system for the conservation of gas in an ON condition;
FIG. 7 is a lower perspective view of the inflation detection system again in an ON condition;
FIG. 8 is a view in side elevation of the inflation detection system in an OFF condition;
FIG. 9 is a top plan view of an automatic system for the conservation of gas as disclosed herein with the cover portion and the retained inflation detection system removed;
FIG. 10 is a bottom plan view of the automatic system for the conservation of gas of FIG. 9;
FIG. 11 is a top plan view of an alternative automatic system for the conservation of gas according to the invention;
FIG. 12 is an amplified top plan view of the automatic system for the conservation of gas of FIG. 11;
FIG. 13 is a bottom plan view of the automatic system for the conservation of gas of FIG. 11;
FIG. 14 is a view in side elevation of the automatic system for the conservation of gas of FIG. 11;
FIG. 15 is an anterior perspective view of the automatic system for the conservation of gas of FIG. 11;
FIG. 16 is a perspective view of a filter and one-way inspiratory valve for the automatic system for the conservation of gas of FIG. 11;
FIG. 17 comprises schematic top plan and side elevation views of another automatic system for the conservation of gas according to the invention; and
FIG. 18 comprises schematic top plan and side elevation views of still another automatic system for the conservation of gas according to the invention.

### DETAILED DESCRIPTION OF PREFERRED EMBODIMENTS

The automatic system and method for the conservation of oxygen and other substances disclosed herein are subject to a wide variety of embodiments. However, to ensure that one skilled in the art will be able to understand and, in appropriate cases, practice the invention disclosed herein, certain preferred embodiments of the broader invention are described below and shown in the accompanying drawing figures.

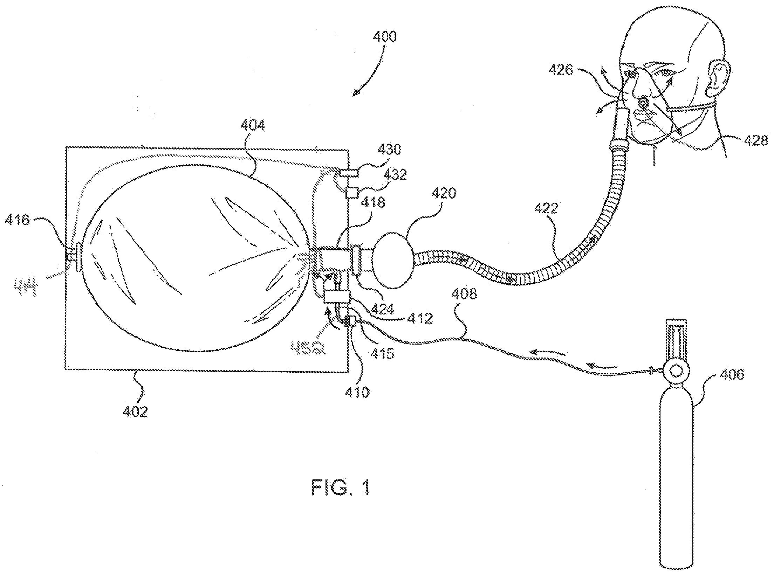

Looking more particularly to the drawings, the structure and operation of an automatic gas conservation system 400 according to the present invention can be understood with reference to FIG. 1. As shown and described herein, the automatic gas preservation system 400 provides an on-demand supply of oxygen at ambient pressure to a recipient, such as a patient breathing mask 426, from a donor reservoir 404. The donor reservoir 404 retains oxygen at ambient pressure and is continually supplied with oxygen from an oxygen source 406, such as a tank of compressed oxygen gas or liquid oxygen. With the donor reservoir 404 retaining oxygen at ambient pressure, a full and ample supply of oxygen is constantly available for patient inspiration. Concomitantly, oxygen losses during patient expiration are substantially eliminated thereby conserving the supply of oxygen without compromising availability to the individual recipient since oxygen within the donor reservoir 404 is automatically replenished.

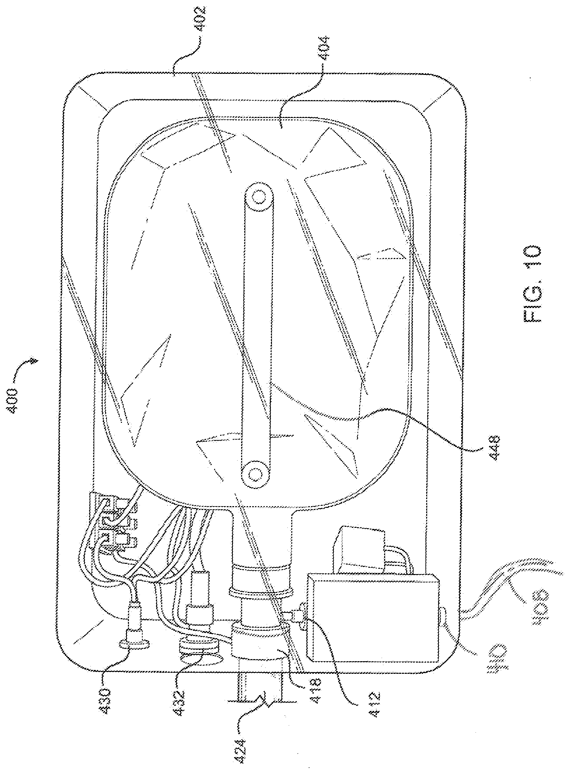

The donor reservoir 404 in this embodiment comprises an expandable and compressible shell, bladder, or other expandable and compressible body that is disposed within a housing 402, which could be a primary housing or a sub-housing within a larger structure. However, the donor reservoir 404 need not necessarily be within a housing 402 to be within the scope of the invention. The housing 402 defines boundaries for the reservoir 404 so that the shell of the reservoir 404 presses toward one or more portions of the boundary defined by the housing 402 as the reservoir 404 is expanded. In this non-limiting example, the housing 402 has a bottom that defines a lower boundary for the reservoir 404, a top that defines an upper boundary for the reservoir 404, and distal ends that define longitudinal boundaries for the reservoir 404. Here, the reservoir 404 has an oblong, egg shape, and the housing 402 has a general cube shape, but other shapes and combinations of shapes are readily possible and within the scope of the invention except as it might be expressly limited by the claims. As in the embodiment of the automatic gas preservation system 400 shown in FIG. 10, for example, the lower wall portion of the shell of the reservoir 404 can be adhered or otherwise secured to the bottom of the housing 402, such as by an adhesive strip 448 or in any other manner.

In this example, the reservoir 404 is defined by first and second oblong panels joined along their edges in a sealed manner to define the shell or outside wall structure with a body portion and a neck. The reservoir 404 is sealed but for an entry orifice in the neck of the reservoir 404. The shell is formed from a flexible and substantially gas impermeable material with it being known to one of skill in the art that numerous such materials are possible, each within the scope of the invention. The shell of the reservoir 404 could, for example, be formed from a flexible polymeric material with or without a lining layer. The material defining the reservoir 404 could, for example, comprise a foil formed by one or more layers of polymeric material with an aluminum lining. Other formations of the reservoir 404 are possible and within the scope of the invention. The reservoir 404 can have combinations including one or more flexible walls, rigid walls, compressible walls, collapsible walls, expandable walls, thin walls, or other walls capable of keeping a volume gas inside.

Preferably, as is enabled by formation of the reservoir 404 of a lightweight, flexible foil, the reservoir 404 once expanded tends to substantially maintain an expanded shape and configuration, whether by its own structural integrity or otherwise, even when it is open to ambient pressure, such as by a fluidic connection to the recipient 426 through ambient pressure tubing 422. As taught herein, when expanded, the reservoir 404 in preferred embodiments does not significantly collapse on its own due to the weight of its walls. When filled with oxygen, the reservoir 404 thus temporarily stores a compartmented volume of oxygen at ambient pressure waiting to be drawn therefrom by the recipient 426.

A fluidic connector 418, which in this example comprises a T-shaped connector, has a first, longitudinal port in fluidic communication with the donor reservoir 404, such as through the aperture in the neck of the reservoir 404. The fluidic connector 418 has a second, longitudinal port in fluidic communication with the ambient pressure tubing 422 and, through that tubing 422, the recipient 426. Finally, the fluidic connector 418 has a third, lateral port between the first and second openings in fluidic communication with the oxygen source 406. The fluidic communication from the source 406 to the connector 418 could, for instance, be through high-pressure tubing 408 acting as a supply conduit from the oxygen source 406 to an oxygen connector 410 fixed to the housing 402 and high-pressure tubing 452 from the oxygen connector 410 to a supply valve 412. The first, second, and third ports are in fluidic communication with one another within the fluidic connector 418.

The supply valve 412, which in this example comprises an electromechanical solenoid valve 412, has an open condition and a closed condition. The valve 412 is fluidically interposed between the pressurized oxygen source 406 and the reservoir 404. When the supply valve 412 is in the open condition, oxygen can be passed from the oxygen source 406, through the tubing 408, through the valve 412, through the connector 418, and into the reservoir 404. When the valve 412 is in the closed condition, the passage of oxygen between the oxygen source 406 and the reservoir 404 is prevented.

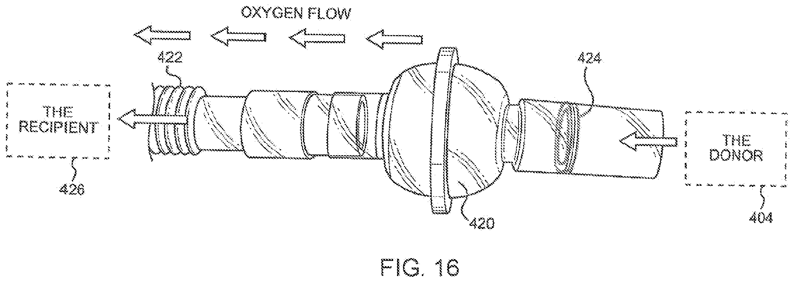

A one-way inspiratory valve 424 is interposed between the reservoir 404 and the recipient 426, such as by being fluidically connected to the second port of the fluidic connector 418 and with the fluidic connector 418 fluidically connected through its first port to the neck of the reservoir 404. The one-way inspiratory valve 424 is operative to enable gas to flow from the donor reservoir 404, through the ambient pressure tubing 422, and to the recipient 426 but to prevent reverse gas flow, such as from the recipient 426 and into the donor reservoir 404. A gas filter 420 is fluidically interposed between the recipient 426 and the one-way inspiratory valve 424 and thus between the recipient 426 and the donor reservoir 404. The filter 420 and the one-way inspiratory valve 424 are shown apart from the remainder of the automatic gas conservation system 400 in FIG. 16.

As disclosed herein, the volume of oxygen in the donor reservoir 404 is retained substantially at ambient pressure. Ambient pressure can be defined as the pressure of the air surrounding the donor reservoir 404. As a recipient undergoes the inspiratory phase of breathing, oxygen will be drawn from the donor reservoir 404 through the ambient pressure tubing 422 thereby drawing from and tending to reduce the volume of oxygen in the donor reservoir 404. Due to the compressible nature of the donor reservoir 404, the reservoir 404 will tend to contract. When it does contract, the donor reservoir 404 is automatically replenished with oxygen by operation of an inflation detection system without pressurization of the reservoir 404 so that the oxygen within the reservoir 404 remains substantially at ambient pressure.

The inflation detection system has a first condition wherein replenishing oxygen is not supplied to the donor reservoir 404 and a second condition wherein replenishing oxygen is supplied to the donor reservoir 404. The first condition can be a condition wherein the donor reservoir 404 is inflated with oxygen to a certain, predetermined state of inflation, and the second condition can be a condition wherein the donor reservoir 404 is inflated with oxygen below the predetermined state of inflation. The inflation detection system is operative to detect when the donor reservoir 404 has reached the predetermined state of inflation. The predetermined state of inflation can be detected when the donor reservoir 404 reaches a predetermined size or other inflation condition in any dimension or combination of dimensions. In embodiments of the invention, the donor reservoir 404 can be considered to have a fully inflated condition, and the inflation detection system detects when the donor reservoir 404 is inflated to the fully inflated condition or to within a predetermined range of the fully inflated condition. By way of example and not limitation, the inflation detection system can detect when the donor reservoir 404 is inflated with oxygen at or above a threshold inflation level, which may be equal to or less than the fully inflated condition.

Made aware of the present invention, one skilled in the art may appreciate plural mechanisms that would operate as inflation detection systems to detect when the donor reservoir 404 is inflated to the predetermined state of inflation. Each such mechanism is within the scope of the invention except as it may be expressly limited by the claims. Inflation detection mechanisms could comprise mechanical systems, electrical systems, electromagnetic systems, optical systems, electro-mechanical systems, sound-activated systems, movement sensors, light sensors, and any other type of system effective to detect when the donor reservoir 404 is inflated to a predetermined state of inflation with it again being noted that the predetermined state of inflation may be reached while the oxygen within the donor reservoir 404 is substantially at ambient pressure.

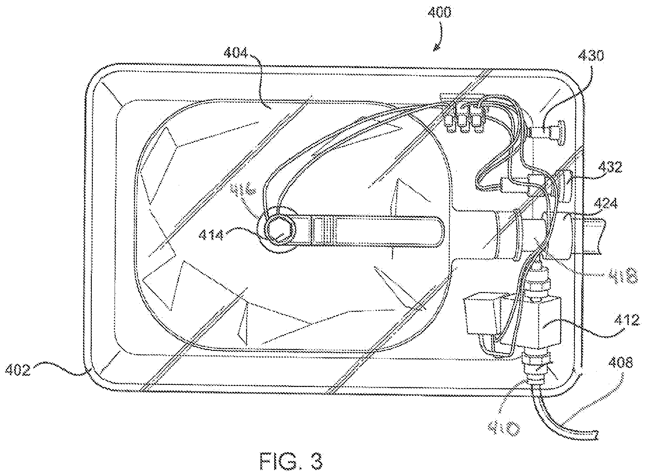

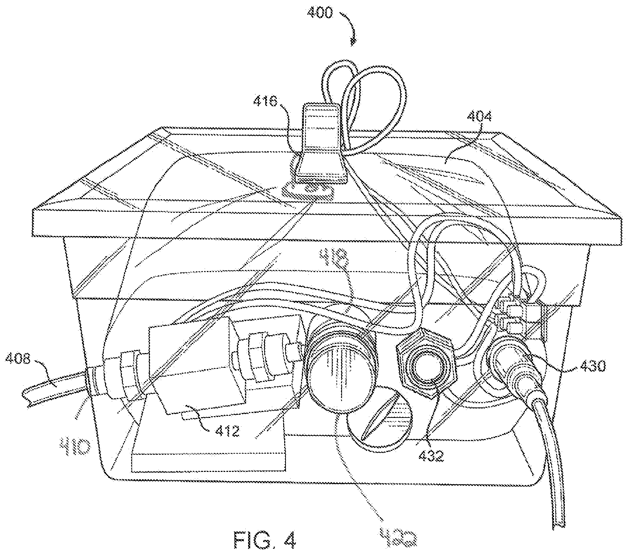

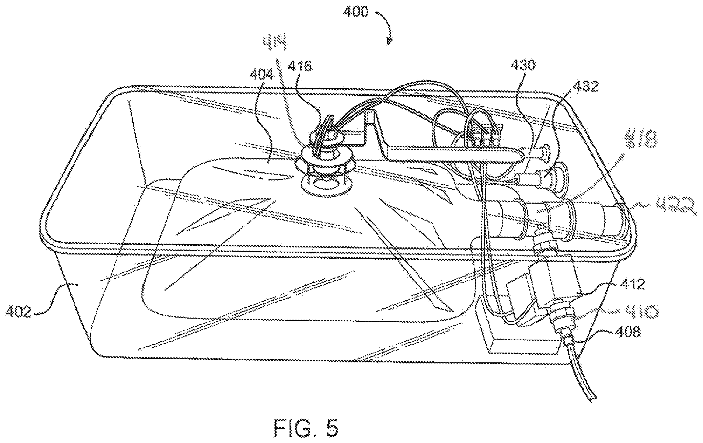

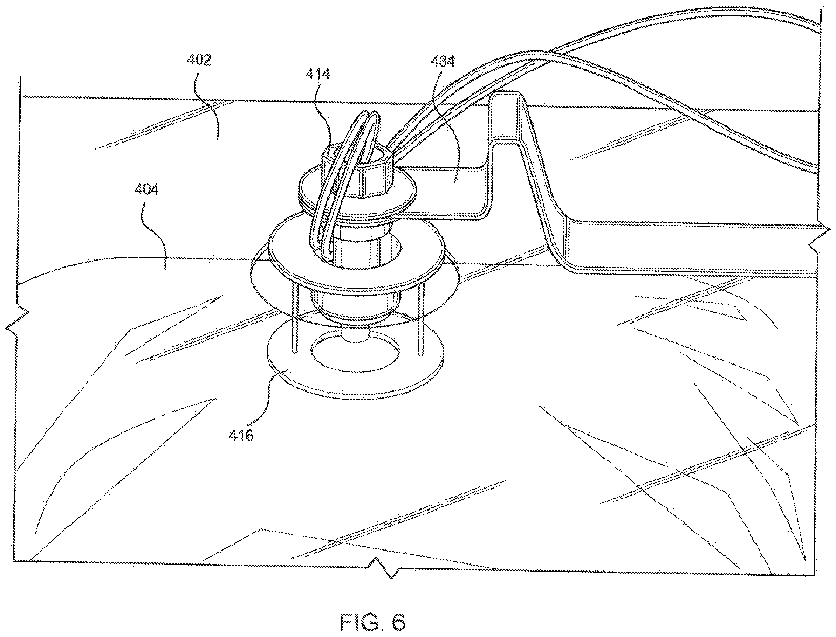

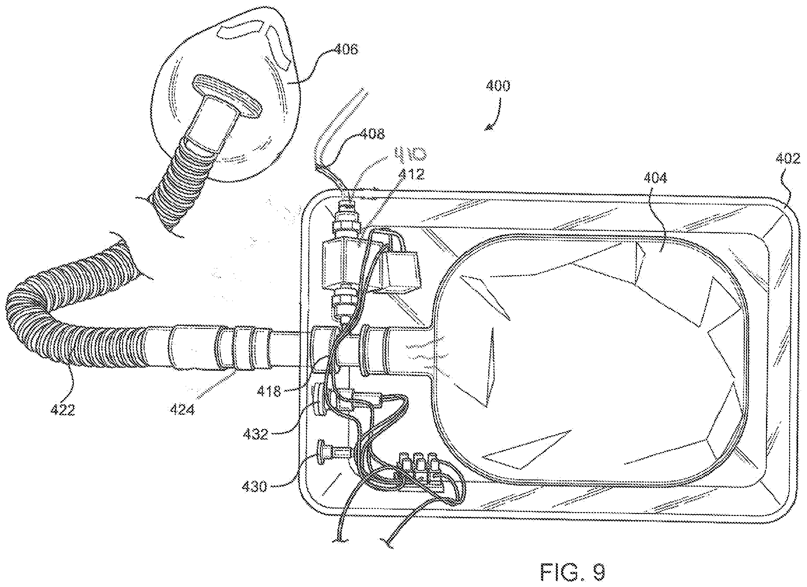

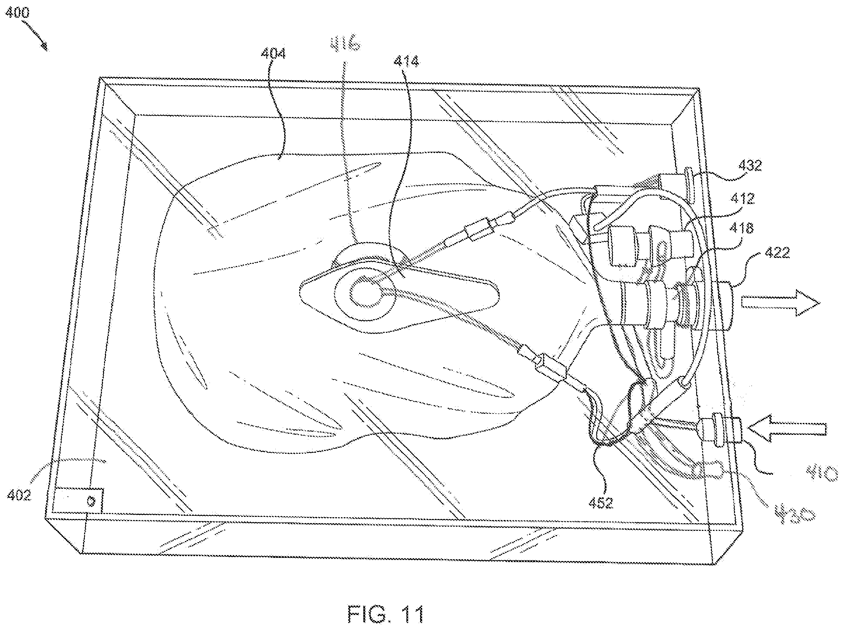

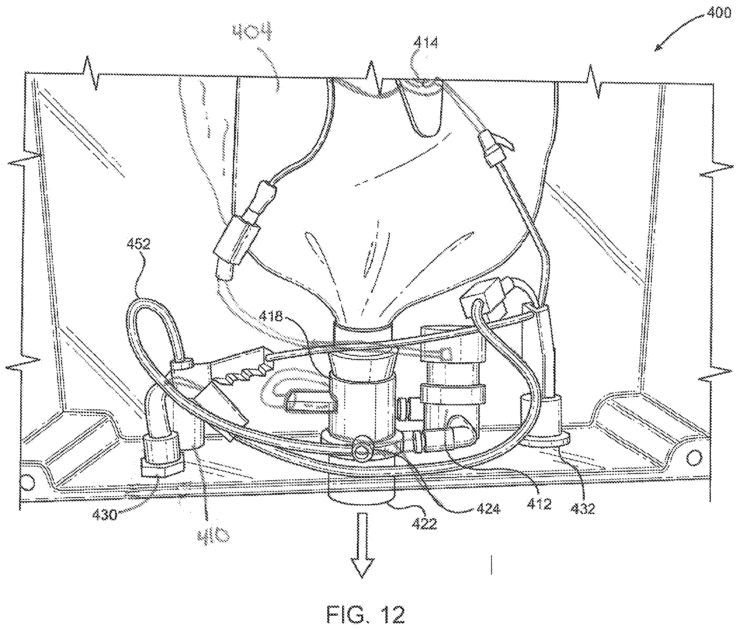

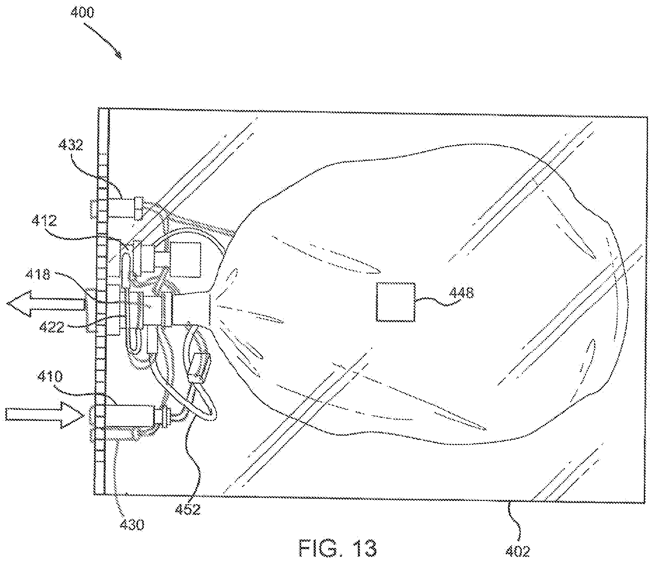

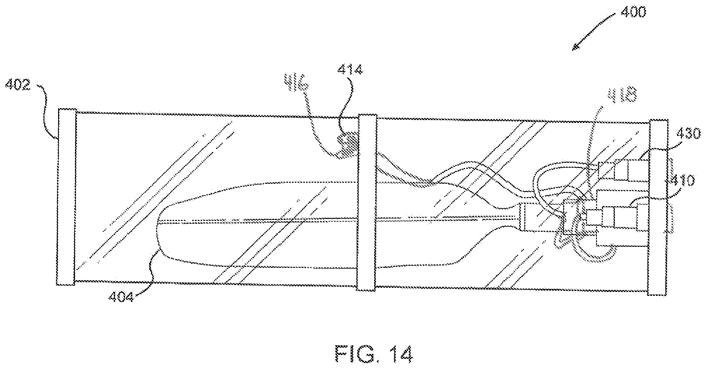

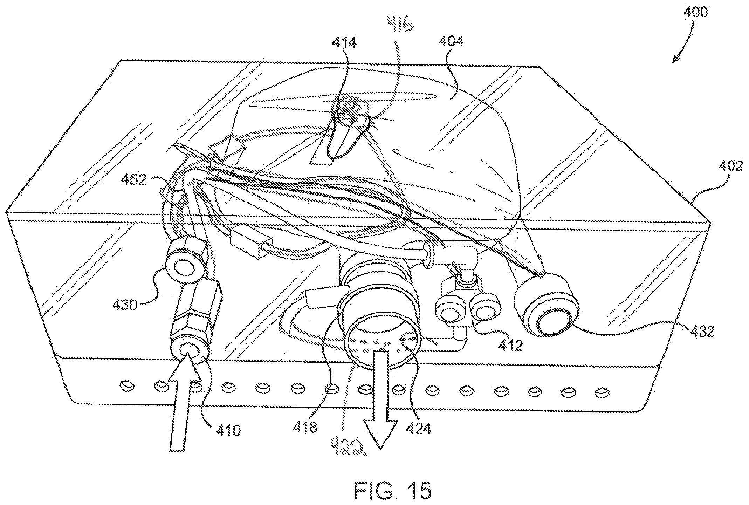

In the non-limiting embodiment of FIG. 1, the inflation detection system comprises an electro-mechanical system for detecting when the donor reservoir 404 is filled to the predetermined state of inflation. The inflation detection system has a contact structure 416 disposed to contact, to be contacted by, to be moved by, or otherwise to be actuated by the donor reservoir 404 when the reservoir 404 reaches a stage of inflation. Within the scope of the invention, the location and construction of the contact structure 416 could vary. In the embodiment of FIG. 1, for instance, the contact structure 416 is disposed to project from or through the distal end wall of the housing 402 and into the inner volume of the housing 402 so that it projects toward and can engage the distal end of the reservoir 404. In the embodiments of FIGS. 3 through 15, however, the contact structure 416 is disposed to project from or through the upper wall of the housing 402 and into the inner volume of the housing 402 to engage a midportion of the reservoir 404. There, the contact structure 416 is retained by a support structure 434 that is fixed to the upper wall of the housing 402. According to the invention, the contact structure 416 could be otherwise retained.

The contact structure 416 is positioned to be moved by the donor reservoir 404 as the reservoir 404 expands toward an inflated condition. The contact structure 416 can, for instance, be depressed, pivoted, rotated, or otherwise actuated by the donor reservoir 404 and more particularly by an expansion of the donor reservoir 404. The contact structure 416 operates as or as a component of or to actuate a flow switch 414. When the contact structure 416 is actuated by the expansion of the donor reservoir 404, the flow switch 414 is caused to actuate the valve 412 between the ON condition where oxygen is permitted to flow from the oxygen source 406 to the reservoir 404 to replenish and fill the reservoir 404 and the OFF condition where oxygen is prevented from flowing from the oxygen source 406 to the reservoir 404. The contact structure 416 is biased, such as by spring force, under the force of gravity, by resiliency, or any other biasing method or combination thereof toward the donor reservoir 404.

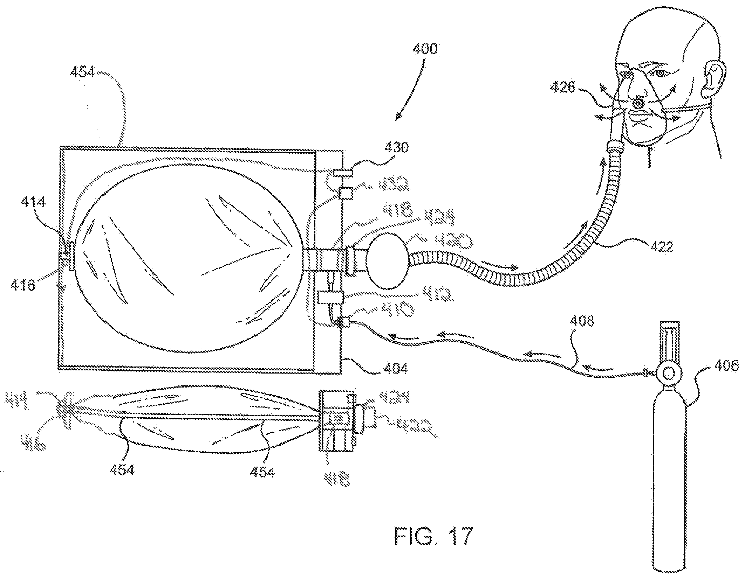

In the non-limiting embodiment of FIG. 1, the donor reservoir 404 is disposed within a housing 402. Additionally or alternatively, the donor reservoir 404 could be disposed within a sub-housing that, in tum, could be disposed in the housing 402 or that could stand independently. Still further, as is shown in FIG. 17, for example, the donor reservoir 404 could be disposed without a housing or enclosure, in which case the contact structure 416 and potentially the flow switch 414 described further hereinbelow could be otherwise retained, such as by a surrounding band, a rigid arm, or another retaining structure 454, for contact or other sensing or engagement relative to the donor reservoir 404. The contact structure 416 and the flow switch 414 could be retained together, potentially as a unit, or in separate dispositions. In FIG. 17, the contact structure 416 is retained by the retaining structure 454, which could be a rigid support arm or any other retaining structure, to engage the donor reservoir 404, and the flow switch 414 is integrated with the contact structure 416.

The contact structure 416 is thus retained by the housing 402, by the retaining structure 454, or otherwise to contact the reservoir 404. Without limiting the invention, the contact structure 416 could be retained to contact the reservoir 404 by being secured partially or entirely within the housing 402 or through an aperture in the housing 402 or through an aperture in a sub-housing that retains the reservoir 404, or the contact structure 416 could be retained to contact a donor reservoir 404 that is not in a housing at all.

The flow switch 414 has an activated state, which may be considered to be the ON condition, when the contact structure 416 is sufficiently moved, such as by extension, pivoting, or other movement, in an inward direction toward the inner volume of the donor reservoir 404. The contact structure 416 is permitted to move inwardly in the direction toward the donor reservoir 404 to the activated state when the volume of oxygen in the donor reservoir 404 falls below the predetermined state of inflation such that the outside wall is or can be deflected or moved inwardly. The flow switch 414 has a deactivated state, which may be considered to be the OFF condition, when the contact structure 416 is moved, such as by retraction, pivoting, or other movement in an outward direction away from the donor reservoir 404. The contact structure 416 is moved outwardly to adjust the flow switch 414 to the deactivated state, which is the OFF condition, when the volume of oxygen in the donor reservoir 404 reaches the predetermined state of inflation to cause the outside wall of the donor reservoir 404 to be advanced outwardly by the expansion of the donor reservoir 404. For instance, where the contact structure 416 is a depression switch, expansion of the donor reservoir 404 will press the outer wall or shell of the donor reservoir 404 outwardly to press the contact structure 416 and the flow switch 414 to the deactivated state.

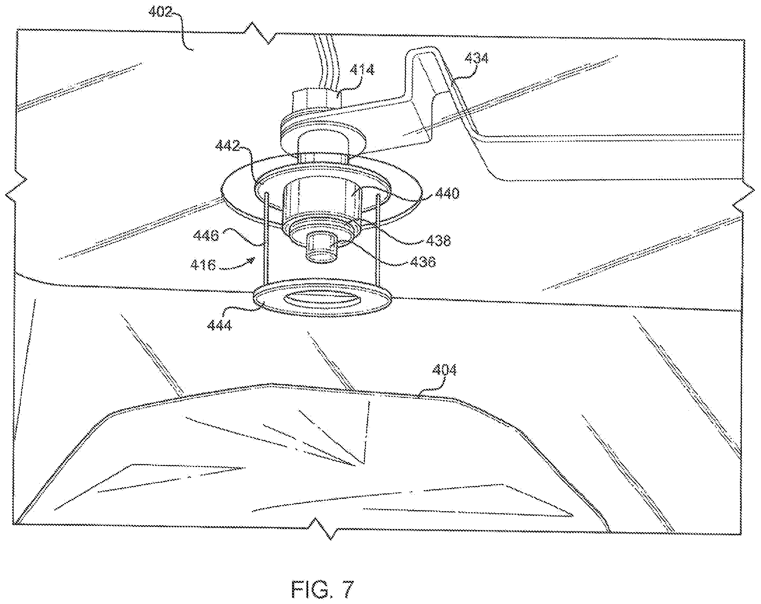

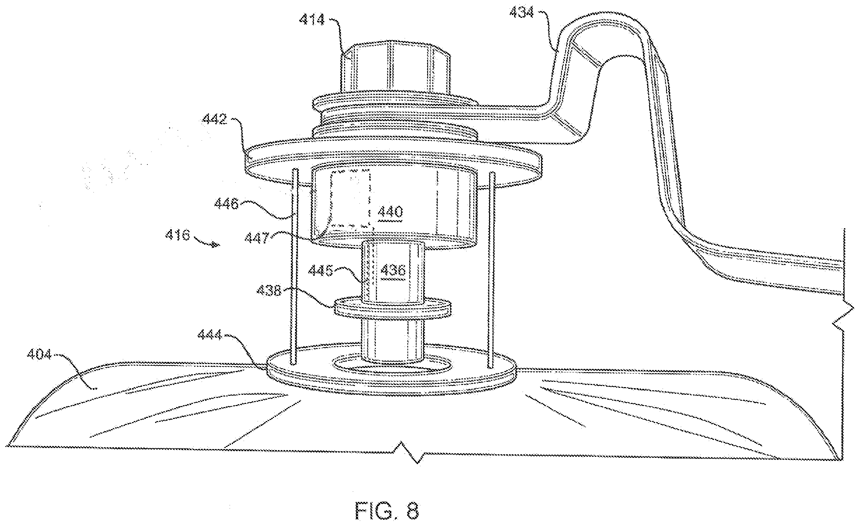

In the embodiment of FIGS. 3 through 10, the contact structure 416 and the switch 414 are embodied as a float switch with an actuation framework. With particular reference to FIGS. 7 and 8, the actuation framework of the contact structure 416 can be seen to be retained to be movable along a vertical axis generally perpendicular to a longitudinal of, and generally the surface of, the donor reservoir 404. The actuation framework of the contact structure 416 has a distal, flat toroidal ring 444 disposed to engage and be engaged by the wall of the reservoir 404. A proximal toroidal ring 442 is maintained in parallel spaced relation to the distal toroidal ring 444 by a plurality of rod members 446, and a collar 440 is fixed to move with the proximal toroidal ring 442.

The actuation framework so formed by the toroidal rings 442 and 44, the rod members 446, and the collar 440 is extendable and retractable relative to a central column 436. An annular plate 438 is fixed along the length of the central column 436 distal to the collar 440 of the actuation framework so that the annular plate 438 retains the contact structure 416 in a floating manner. As FIG. 7 shows, the collar 440 tends to drop into contact with the annular plate 438 under the natural force of gravity when the donor reservoir 404 is filled below a given level.

The central column 436 houses a magnetic switch 414, such as a reed switch 414, and the floating actuation framework of the contact structure 416 retains a magnet 447 within the collar 440. When the actuation framework is extended as in FIG. 7 where the donor reservoir is below the state of inflation, the contacts 445 of the reed switch 414 (shown in FIG. 8) are attracted into contact with one another to complete the electrical circuit and trigger the switch 414 to an activated condition, which actuates the valve 412 to the ON condition where oxygen is permitted to flow from the oxygen source 406 to the reservoir 404. When the reservoir 404 is filled to the predetermined state of inflation, the magnet 447 within the collar 440 is moved away from the contacts 445 of the reed switch 414 to break the circuit and trigger the switch 414 to a deactivated condition, which actuates the valve 412 to the OFF condition wherein oxygen is prevented from flowing from the oxygen source 406 to the reservoir 404.

By virtue of the biasing of the contact structure 416, which can be by any mechanism including gravity, a resiliently compressible or extendible member, or any combination of mechanisms, the contact structure 416 automatically moves to the activated state to actuate the flow switch 414 and the valve 412 to the ON condition to permit oxygen to flow from the oxygen source 406 to the reservoir 404 when the volume of oxygen in the donor reservoir 404 falls below the predetermined threshold value, such as below the predetermined state of inflation. When the volume of oxygen in the donor reservoir 404 reaches the predetermined threshold value, such as at or above the predetermined state of inflation, the contact structure 416 is moved by the wall of the donor reservoir 404 to the deactivated state, and the switch is disposed in the OFF condition. In the deactivated state, the valve 412 is closed to prevent the flow of preservative gas from the source 406 to the donor reservoir 404.

The donor reservoir 404 can thus be inflated, such as to or within a given range of the maximum volume of the donor reservoir 404 without over-inflation or pressurization of the donor reservoir 404. Oxygen within the donor reservoir 404 is thus prevented from exceeding approximately ambient pressure. Except as might otherwise be required by the claims, however, embodiments of the invention could calibrate the contact structure 416 or the flow switch 414 or both to be induced to the deactivated state at some other predetermined inflation condition or pressure, including potentially a pressure or inflation condition in excess of ambient pressure or to some inflation condition well below the maximum volume of the donor reservoir 404. The flow switch 414 and the valve 412 can be electrical, mechanical, electro-mechanical, or otherwise configured and constructed.

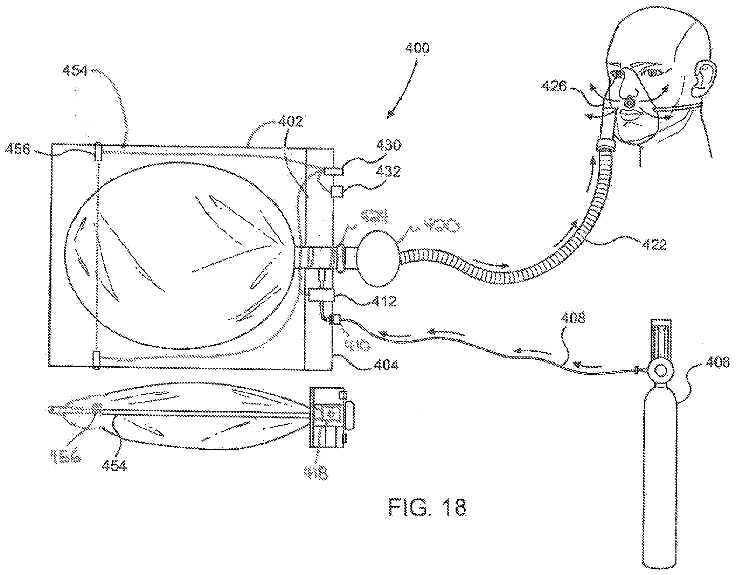

It will again be observed that one skilled in the art would appreciate other mechanisms that would operate as inflation detection systems to detect when the donor reservoir 404 is inflated to the predetermined state of inflation with each such mechanism being within the scope of the invention except as it may be expressly limited by the claims. For instance, as in the embodiment of FIG. 18, for instance, the inflation detection system could alternatively take the form of a contactless detection system 456, such as an optical detection system that could be carried forth by, for instance, a laser detection system, a camera system, an infrared inflation detection system, or any other effective optical or contactless detection system. In the non-limiting embodiment of FIG. 18, for example, a contactless detection system 456 is formed with a light emitter, such as a laser or other light emitter, retained to one side of the reservoir 404 and a light receptor disposed to the opposite side of the reservoir 404. Under such constructions, the inflation condition of the donor reservoir 404 can be sensed in a contactless manner, such as where the donor reservoir 404 is inflated to a condition where the reservoir 404 prevents the communication of light from the light emitter to the light receptor, where the reservoir 404 demonstrates a predetermined reflectance value, or in some other contactless manner.

In the embodiments of the automatic gas conservation system 400 of FIGS. 1 through 16, the supply valve 412 comprises a solenoid valve that is in electrical communication, such as through electrical wiring in an electrical circuit, with the flow switch 414. As illustrated, an electrical control system, which can include electrical circuitry, electronic memory, wiring, and other electrical control and connection components, cooperates with the inflation detection system to induce the solenoid supply valve 412 to an open condition to permit the flow of oxygen from the source 406 when the flow switch 414 is in the activated state. The electrical control system can receive power from a power source, which could be a source of alternating current through a power supply connection 430, a source of direct current such as a battery power source, or some other source of electric power. The flow of electrical power from the power source can be controlled by a power switch 432. The solenoid valve 412 is induced by the inflation detection system and the electrical control system to a closed condition to prevent the flow of oxygen from the source 406 to the reservoir 404 when the flow switch 414 is in the deactivated state. Each of the components referenced herein can be further combined or separated within the scope of the invention.

The solenoid valve 412 can be electrically opened when the electrical circuit is closed by the movement or other actuation of the flow switch 414 of the inflation detection system to the activated condition. The solenoid valve 412 is automatically closed to prevent further filling of the donor reservoir 404 when the electrical circuit is opened by the contact structure 416 and the flow switch 414 is moved to the deactivated condition, which can be indicative that the donor reservoir 404 is filled to the predetermined state of inflation. In the example of the invention where the contact structure 416 and the flow switch 414 are actuated by a pressing or pushing of the contact structure 416 outwardly by the reservoir 404, an open electrical circuit is established where no electricity flows when the contact structure 416 is sufficiently pressed outwardly by the reservoir 404 and the solenoid valve 412 is in a closed position. When the contact structure 416 is sufficiently advanced, such as by extension inwardly toward the reservoir 404, indicating that the reservoir 404 has fallen below the predetermined state of inflation, the electrical circuit is closed to permit the flow of electricity to actuate the solenoid valve 412 to an open condition so that oxygen can flow to fill the donor reservoir 404.

Even when the valve 412 is in an open condition, the rate of flow, the pressure of flow, or both the pressure and rate of flow of oxygen from the source 406 to the donor reservoir 404 can be limited, such as by a flow-limiting connector 415 as shown, for instance, in FIG. 1. The flow-limiting connector 415 could limit the flow rate of oxygen from the source 406 to the donor reservoir 404 to a predetermined flow rate, such as less than 1 liter per minute or any other flow rate. The flow-limiting connector 415 could, for example, comprise a narrow-diameter tube connector, such as a connector having an inner diameter of 0.02 mm or some other dimension reduced as compared to other conduit connections within the fluidic system. Rapid changes in pressure within the donor reservoir 404 can thus be prevented on opening of the valve 412.

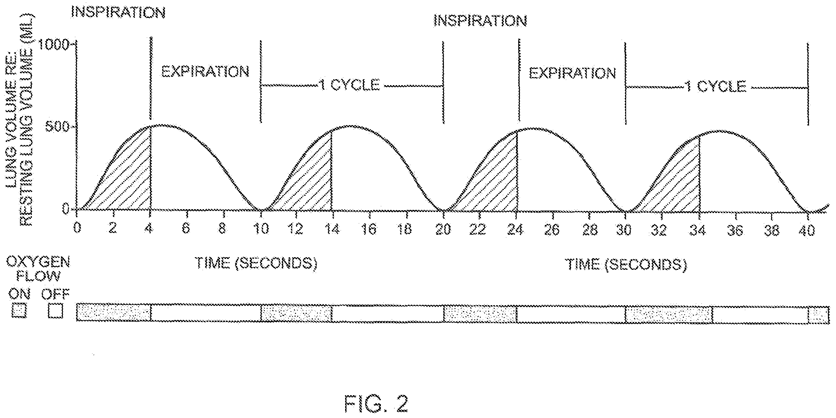

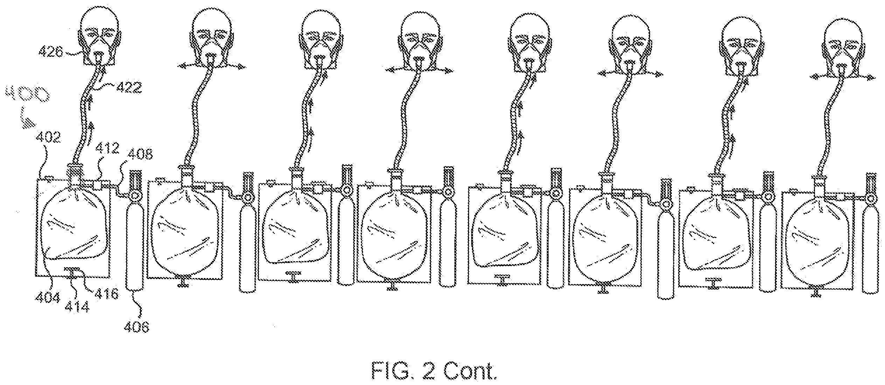

Referring to FIG. 2, the automatic gas conservation system 400 is depicted in operation during a series of respiratory cycles to provide an on-demand supply to a recipient 426, such as a mask worn by a human or other living patient in need. In operation of the automatic gas conservation system 400, inspiration by the patient will operate to draw oxygen at ambient pressure from the donor reservoir 404 thereby tending to contract the reservoir 404. When the reservoir 404 falls between the predetermined state of inflation, the reservoir 404 is automatically filled to the predetermined state of inflation by a supply of oxygen from the source 406. A volume of continually-replenished oxygen at ambient pressure is thus available within the reservoir 404 to be drawn through the one-way inspiratory valve 424 and the ambient pressure tubing 422 during a natural inspiration phase of a breathing cycle. When the recipient 426 is not engaged in inspiration, no oxygen is drawn from the reservoir 404. When the volume of oxygen within the reservoir 404 falls below the predetermined state of inflation, the inflation detection system formed by the contact structure 416 and the flow switch 414 will detect the same and trigger the valve 412 to an open condition. Flow of oxygen is then permitted from the oxygen source 406 so that the donor reservoir 404 will be filled with oxygen until the predetermined state of inflation is reached. When the predetermined state of inflation is reached, the inflation detection system will detect the same and trigger the valve 412 to a closed condition to prevent the further supply of oxygen to the donor reservoir 404 from the source 406 until a further inspiration phase of a breathing cycle draws a volume of oxygen from the reservoir 404. The donor reservoir 404 is thus automatically supplied with oxygen while pressurization of the oxygen in the reservoir 404 is automatically prevented. Supplemental oxygen is safely and effectively supplied to the patient at ambient pressure in an on-demand volumetric displacement system enabling the transfer of oxygen during the entire inspiratory phase of the breathing cycle while the wasteful release of oxygen during the expiratory phase of breathing, indeed at any phase other than the inspiratory phase, is prevented.

The donor reservoir 404 automatically receives replenishing oxygen from the pressurized source 406 through the high-pressure tubing 408 and through the supply valve 412 as soon as the reservoir 404 begins to collapse. The automatic refilling of the reservoir 404 ensures that the donor reservoir 404 always retains a supply of oxygen available for the next inspiratory phase of the breathing cycle while the oxygen in the reservoir 404 never exceeds ambient pressure. Where the donor reservoir 404 is visually exposed, such as through a partially or completely transparent housing 402 or an observation aperture in the housing 402, an observer is provided with visual confirmation of the state of inflation of the donor reservoir 404. The automatic gas conservation system 400 can thus provide a synchronized delivery of supplemental oxygen to a recipient 426 as the donor reservoir 404 and the system 400 in general synchronize with the physiological ventilations of a patient based on the storage and replenishment of oxygen in the donor reservoir 404 at ambient pressure and the termination of the supply of oxygen automatically on the donor reservoir 404 reaching the predetermined state of inflation.

Within the scope of the invention, the system 400 can measure, record, and analyze the flow of oxygen and the breathing characteristics of a patient. By way of non-limiting example, a volumetric measuring flow meter could be connected to the source 406 of oxygen. Additionally or alternatively, one or more flow meters could be retained within the housing 402 along the path of gaseous flow through the system 400. For instance, a flow meter could be disposed to measure oxygen passing through the valve 412. In the depicted embodiments, the valve 412 can incorporate a flow meter such that the same should be considered to be illustrated therewithin, or a flow meter could be otherwise disposed. For instance, a flow meter could further or alternatively be disposed between the reservoir 404 and the ambient pressure tubing 422. By measuring the volume of oxygen supplied to a recipient 426 by the system 400, such as over a given time period, per cycle of inspiration and expiration, or otherwise, plural determinations, measurements, and analyses can be made. For instance, one can determine the volume of oxygen inspired by the patient and, additionally or alternatively, the volume of oxygen remaining in the oxygen source 406. Through electronic memory and software operating on the electrical system or in communication therewith, the system 400 can harvest, process, and analyze data based on usage of the system 400.

As often shown and described herein, the recipient 426 can be the breathing mask of a living patient receiving supplemental oxygen, but other recipients and delivery equipment are possible and within the scope of the invention. When worn by a patient, the patient and the breathing mask or other oxygen delivery equipment may collectively be referred to as the recipient 426. Other recipient delivery equipment could, for example, comprise other respiratory accessories, such as but not limited to nasal cannulas, laryngeal mask airways (LMA), endotracheal tubes, tracheostomys, ventilator attachments, CPAP machine connectors, Ambu bags, or even delivery devices for recreational oxygen. The automatic gas conservation system 400 is not limited with respect to the recipient 426 unless the claims expressly so require.

As shown in FIG. 1, a recipient mask 426 can have one or more one-way expiratory valves 428 and can include adjustment mechanisms as is known to the art for adjusting oxygen supply to the patient. As necessary, the concentration of oxygen that the patient needs as determined by the physician can be reliably and predictably diluted and controlled with devices currently in use and that are within the scope of the system 400. By way of example and not limitation, the number, diameter, or other characteristic of orifices in the inspiration tube 422 or the recipient mask 426 can be adjusted to allow more or less oxygen to achieve the desired concentration to the recipient mask 426 for the patient as clinically needed.

With further reference to FIG. 2, the method for the necessary supply of oxygen to a patient recipient 426 and the synchronized operation of the automatic gas conservation system 400 in relation thereto can be further understood. There, the dynamics of the breathing cycle are depicted in parallel with the filling and refilling operations of the donor reservoir 404 of the automatic gas conservation system 400. To expand the lungs, the inspiratory muscles overcome two key factors, namely, compliance of the lungs and airway resistance mainly in the form of frictional resistance to the flow of air through the airways. At the start of inspiration, the diaphragm contracts and descends, expanding the thoracic volume. The descent of the diaphragm compresses the abdominal contents and decompresses the contents of the thoracic cavity. With expansion of the thoracic cavity and its decompression, both intrapleural pressure and alveolar pressure decrease. Alveolar pressure decreases to a sub-atmospheric level, and the pressure gradient for the flow of air into the lungs is established. Air flows into the lungs and lung volume increases until the alveolar pressure rises to the atmospheric level (0 cm H₂0) when the pressure gradient for flow of air into the lungs ceases to exist. At the end of quiet inspiration, intrapleural pressure reaches about -8 cm H₂O, and the transpulmonary pressure distending the lungs increases to 8 cm H₂O (P1 = Pa-Ppl = 0 - (-8) = 8 cm H₂O).

During quiet expiration, the cycle is reversed. The inspiratory muscles relax, and the inward elastic recoil of the lungs results in deflation of the lungs. During deflation, the lungs and chest wall move as one unit. Airflow out of the lungs ceases when alveolar pressure equals atmospheric or ambient pressure (0 cm H₂0).

Based on Boyle's law, in a closed system where the number of gas molecules is constant, at any constant temperature, the pressure exerted by a gas varies inversely with the volume of the gas. Therefore, as the volume of a gas increases, the pressure exerted by the gas decreases. Conversely, the pressure increases as the volume decreases.

Accordingly, in operation of the present system 400 and method, when a patient takes a breath during the inspiratory phase of the breathing cycle, a continuous flow of supplemental oxygen enters the patient's lungs from the system 400 throughout the entire inspiratory phase of the breathing cycle. The flow rates, pressures, and volumes are different at different points of the inspiratory phase. The flow starts by a drop in alveolar pressure below ambient pressure inside the donor reservoir 404 with it being again recognized that the system 400 could work with higher and lower pressures than ambient unless the claims require otherwise. Then, the donor reservoir 404 supplies non-pressurized oxygen at ambient pressure directly to the patient through the recipient 426 as a continuous flow but at different speeds during the inspiratory cycle. The flow rates, pressures, volumes, and respiratory rate are closely synchronized to those of the patient due to the donor reservoir 404 being maintained at ambient pressure. Having a system 400 that matches the supplement of oxygen to a patient's physiological ventilation values at each point in time throughout the inspiratory phase of the breathing cycle ensures reliable delivery of the prescribed oxygen concentration through a recipient facemask 426 or any other oxygen delivery equipment available without supplementing less or more oxygen flow than planned. Flow rate, alveolar pressure, and tidal volume can be synchronized at each point throughout the inspiratory phase of the breathing cycle with it being recognized that the physiological ventilation values of patients are different at different points in the inspiratory phase.

The continuous flow of oxygen towards the patient's lungs is sustained until the patient's intrathoracic pressure is at equilibrium with the ambient pressure of the donor reservoir 404 at the end of the inspiratory phase of the breathing cycle. At that time, the flow of oxygen to the patient stops until the beginning of the next inspiratory phase. No oxygen flows from the system 400 to the patient during the expiratory phase of the breathing cycle, but a flow of oxygen from the source 406 of compressed, high-pressure oxygen is supplied to the cause the donor reservoir 404 to expand until the predetermined state of inflation is reached. Once the reservoir 404 is refilled to the predetermined state of inflation and at ambient pressure, the donor oxygen reservoir 404 is ready to supply supplemental oxygen when the patient's next inspiratory phase begins. The inflation detection system automatically shuts off the supply valve 412 to prevent further oxygen flow once the reservoir 404 is full and at ambient pressure. The passive and sustained transfer of a reliable volume and concentration of supplemental oxygen from the donor reservoir 404 to the patient's lungs throughout the entire inspiratory cycle is possible with the donor reservoir 404 placed between a compressed oxygen source 406 and the patient's oxygen delivery equipment, such as a recipient mask 426.

The automatic gas conservation system 400 can thus be employed to provide supplemental oxygen to patients in a wide variety of circumstances. Furthermore, except as the claims may be expressly limited, the automatic gas conservation system 400 is not limited to handling oxygen, and it is not necessarily limited to providing gas to patients at all. Other applications where the dispensing of gas or other substances with automatic replenishment of the reservoir 404 are possible.

Many conditions may require supplemental oxygen. For instance, at the writing of the present document, many thousands of patients require supplemental oxygen due to acute hypoxemic respiratory failure deriving from the COVID-19 coronavirus disease. Other illnesses requiring supplemental oxygen include acute exacerbations of chronic obstructive pulmonary disease (COPD) and acute severe bronchial asthma. Patients with chronic obstructive pulmonary disease often have chronic hypoxaemia with or without CO₂ retention. Oxygen in this situation is required until the exacerbation is settled. While a high FiO₂ of up to 100% can be initially administered in case hypoxemia is severe, it is soon tapered to around 50-60% FiO₂. The goal of supplemental oxygen is to maintain a PaO₂ (Partial Pressure of arterial Oxygen) of 55-60 mm Hg, which corresponds to SpO₂ of about 90%. Higher concentrations of oxygen blunt the hypoxic ventilatory drive, which may precipitate hypoventilation and CO₂ retention. It is considered preferable to use a regulated flow device such as a venti mask, which guarantees oxygen delivery to a reasonable extent. Once the patient is stabilized, one can shift to nasal prongs, which are more comfortable and acceptable to most patients. Patients with acute severe asthma or status asthmaticus have severe airway obstruction and inflammation. They are generally hypoxemic. With such conditions, an arterial blood sample is immediately obtained, and oxygen is started via nasal cannula or preferably via a facemask at a flow rate of 4-6 L/min to achieve FiO₂ of 35 to 40%. Higher flow is unlikely to improve oxygenation. The flow rate is adjusted to maintain a PaO₂ of about 80 mm Hg or near normal value. Assisted ventilation is required in case there is persistence of hypoxemia and/or precipitation of hypercapnia.

These clinical samples show the importance of supplying reliable FiO₂ (fraction of Inspired Oxygen) to a patient. However, with conventional systems, they also require continuous flow at high flow rates to overcome air entrapment, making these systems wasteful when supplementing directly compressed oxygen from a cylinder to patients. Also, even if the systems intermittently deliver compressed oxygen only during the inspiratory phase of the breathing cycle, such as with pulse flow (PF), to avoid the continuous delivery of oxygen, these systems must provide pulses of compressed oxygen to the patient containing significant more oxygen than the patient requires to overcome air entrapment.

By providing oxygen only on demand during the inspiratory phase of the breathing cycle, the present system 400 and method are elegant and efficient in conserving oxygen and lowering oxygen costs without compromising necessary supply. Since there is no gas delivery to the patient during the expiratory phase of the breathing cycle, the flow of oxygen from the source 406 of compressed oxygen is intermittent during inspiration only and not a continuous flow as demanded by, for instance, high-concentration oxygen masks of the prior art to keep a reliable concentration of oxygen and to overcome air entrapment that otherwise dilutes oxygen concentration and delivers an unreliable concentration to the patient. Oxygen delivery systems using compressed oxygen at a constant flow, particularly at high flow rates, are wasteful and costly. Moreover, the delivery of pressurized oxygen can be complex and difficult, often requiring complicated software, detailed algorithms, and multiple components susceptible to malfunction and breakage thereby requiring repairs and demanding safety mechanisms that further contribute to the cost and complexity of such systems.

Thus, under typical systems of the prior art, a relatively inexpensive oxygen delivery system can be provided, but it demands the constant flow of pressurized oxygen with half or more of the precious gas being simply exhausted to the environment. Systems with oxygen delivered with pulse flow (PF) through a facemask or another oxygen delivery device do seek to supply oxygen only during the inspiratory phase and not during exhalation seeking to reduce total oxygen needs. However, that delivery demands expensive equipment and is not imparted at ambient pressure. Furthermore, providing a pulse of supplemental oxygen properly-timed to synchronize perfectly with the breathing of a patient can be difficult or impossible, particularly where patient oxygen requirements change over time.

The on-demand supply of oxygen to be naturally inspired that is provided by the donor reservoir 404 with the present automatic gas conservation system 400 overcomes numerous deficiencies and limitations exhibited by systems of the prior art. For instance, to achieve the prescribed inspired oxygen concentration, many prior art systems are dependent on the patient's peak inspiratory flow rate (PIFR). For example, when a patient requires low-inspired oxygen concentration, using a nasal cannula at a low flow rate will help, but this practice limits the patient's oxygen only to a low inspired oxygen concentration. Should the patient increase his or her oxygen requirements significantly, the inspiratory effort to drive more air into the lungs, which is dependent on tidal volume, 'speed' of inspiration, and respiratory rate, will make the PIFR exceed the flow rate at which oxygen or an oxygen/air mixture is supplied by the nasal cannula or other delivery device. This will mean that at the time of PIFR more or less entrainment of room air occurs, altering the resulting FiO2 in an unpredictable fashion. On the other hand, if high concentrations of oxygen are needed by a patient, using a non-rebreathing face mask at very high flows of oxygen (10-15L/Min) reassures a reliable delivery of oxygen volume at the prescribe concentration and is less dependent on PIFR. However, half or more of the oxygen is wasted to the environment with supply costs being commensurately increased.

While a compressed gas tank is often depicted and referred to as the oxygen source 406 herein, other oxygen sources 406 are possible within the scope of the invention. By way of further, non-limiting examples, the automatic gas conservation system 400 can provide on-demand oxygen to patients with oxygen supplied by an oxygen concentrator. An oxygen concentrator does not require a tank. Instead, it takes in air and removes the nitrogen from it thereby leaving the oxygen-enriched gas for those patients requiring medical oxygen. The typical flow of this compressed oxygen is 1-5 liters/minute. High-end oxygen concentrators can deliver upwards of 50L/minute, but they require more electricity and more maintenance.

By placing an automatic gas conservation system 400 as disclosed herein between the oxygen concentrator and the recipient 426, such as a patient face mask or a nasal cannula, an excess of oxygen can be stored at ambient pressure for use if, due to flow limitations, volume demands, or otherwise, sufficient supply is not provided by the concentrator. For example, if the oxygen concentrator is providing 10 L/minute and the patient suddenly needs more as his saturation level is dropping, there will be a volume of oxygen at ambient pressure available in the donor reservoir 404. Without the reservoir 404, the patient would be limited to the flow of the concentrator, which itself is limited. Therefore, without the reservoir 404, if a patient needs more oxygen to survive, the choices are to increase oxygen flow to the mask, which may be impossible, or intubate the patient and use mechanical ventilation, something which both doctor and patient want to avoid.

Where the oxygen source 406 is an oxygen concentrator, the automatic gas conservation system 400 can be placed between the oxygen concentrator and the patient mask 426 or other recipient so that, as oxygen leaves the concentrator, it enters the large reservoir 404 where it remains at ambient pressure until the patient inhales. As the patient breathes in and draws oxygen from the reservoir 404, the reservoir 404 begins to deplete, the supply valve 412 from the oxygen concentrator as the oxygen source 406 opens to replenish the reservoir 404 with compressed oxygen from the oxygen concentrator. As the patient exhales, no flow occurs between the reservoir 404 and the patient through the recipient mask 426 or otherwise. Rather than wasting the oxygen flowing from the concentrator during the exhalation phase of the patient, the flow is employed to replenish the reservoir 404. Once the reservoir 404 is full, the supply valve 412 stops the flow of oxygen from the oxygen concentrator source 406. When the patient breathes in again and the donor reservoir 404 contracts to below the predetermined state of inflation, the shut off valve 412 opens to replenish the reservoir 404 with oxygen from the oxygen concentrator, and the cycle repeats with every breath. In this manner, oxygen not taken in by the patient during inspiration is stored rather than lost. In one example, a concentrator 406 with an output of 20L/minute used with a patient needing only about 5 liters of highly concentrated oxygen during inhalation leaves 10 liters or more that could extend supply availability. Oxygen concentrators can thus be used for their intended purpose while having fewer demands with respect to work hours, electricity, wear and tear, and repairs thereby representing a more useful and reliable investment for the end user. Also, the system 400 and the concentrator as the oxygen source 406 cooperate to provide more reliable concentrations of oxygen to patients that require higher concentrations.

As disclosed herein, the automatic gas conservation system 400 and method provide a gas, or a mixture of gases, from the donor reservoir 404 to the recipient 426 at ambient pressure. The gas or a mixture of gases at ambient pressure within the reservoir 404 can be drawn from the donor reservoir 404 when the recipient 426 drops its pressure below that of the donor reservoir 404, and the drawing of ambient pressure gas from the reservoir 404 stops immediately once the pressure of the recipient 426 equilibrates with that of the reservoir 404. The system 400 can provide a gas or a mixture of gases from the donor reservoir 404 to the recipient 426 at ambient pressure, and the percentage of gases in the mix reaching the recipient 426 can be regulated, such as by the resistance placed in the conduit of each gas involved in the mixture at ambient pressure.

The system 400 conserves gas from one or more sources 406 by limiting the flow of a continuous pressurized gas or gases to only when a recipient 426 creates the need for the gas or gases by dropping its pressure below the ambient pressure of the donor reservoir 404. The donor reservoir 404 is thus capable of passively permitting the transfer of a gas or gases from an ambient pressure reservoir 404 by making the gas or gases available to the recipient 426 in a manner that matches the exact volume and speed of the demand based on the control of the pressure difference by the recipient 426. In embodiments of the system 400, the donor reservoir 404 is not only at ambient pressure but it is also large enough to accommodate the transfer in a completely passive way and without resistance of the volume of gas or gases in a 1:1 ratio at every point in time during the transfer from the beginning to the end of the flow created by the pressure difference between the recipient 426 and the donor reservoir 404, such as an inhalation phase during the respiratory cycle. In practices of the system 400, diagrams of the speed, pressure, time, and volume of patient inspiration and gaseous transfer from the donor reservoir 404 are equivalent and will likely be substantial mirror images. The drop in pressure of the recipient 426, such as during inhalation, is entirely used for the transfer of volume from the reservoir 404. No extra pressure is required to open a pressure check valve to start the flow as would be the case with a chamber or reservoir containing oxygen at a higher pressure than ambient pressure. The system 400 can work as a closed system, or it can be open to ambient pressure of the environment while being maintained at ambient pressure. The system 400 conserves gas or gases by limiting the flow of the gas or gases to the recipient 426 only when needed. Since a patient intakes supplemental oxygen flowing to their recipient mask 426 only during inspiration, a far reduced volume of oxygen is required, such as one-half to one-third, as compared to continuous flow systems.

In practices of the invention, the system 400 can be used as a source to provide variable oxygen concentrations for CPAP machines used in the treatment of sleep apnea and COPD. The system 400 can help conserve oxygen from the pressurized source 406 by, for example, connecting the system 400 to the air input of the CPAP machine. The system 400 can also be employed to provide a reservoir 404 at ambient pressure for oxygen concentrators so patients can inhale or inspire a more reliable concentration of oxygen at ambient pressure, especially when high flows are demanded to treat a patient with respiratory insufficiency. Moreover, the system 400 can help oxygen concentrators as sources 406 of oxygen to provide the same oxygen concentration of oxygen to a patient with less required flow of oxygen, decreased hours of operation, reduced electricity consumption, increased longevity to the machine, and fewer repairs and parts. Still further, with the gas conserved. oxygen concentrators that previously supplied just one patient could potentially be used for plural patients concomitantly depending on the required supply rates.

As used herein, references to items in the singular should be understood to include items in the plural, and vice versa, unless explicitly stated otherwise or clear from the text. Grammatical conjunctions are intended to express any and all disjunctive and conjunctive combinations of conjoined clauses, sentences, words, and the like, unless otherwise stated or clear from the context. Thus, for example, the term "or" should generally be understood to mean "and/or." Recitation of ranges of values herein are not intended to be limiting, referring instead individually to any and all values falling within the range, unless otherwise indicated herein, and each separate value within such a range is incorporated into the specification as if it were individually recited herein. The words "about," "approximately," and the like, when accompanying a numerical value, are to be construed as indicating a deviation as would be appreciated by one of ordinary skill in the art to operate satisfactorily for an intended purpose. Similarly, words of approximation such as "approximately" or "substantially" when used in reference to physical characteristics should be understood to contemplate a range of deviations that would be appreciated by one of ordinary skill in the art to operate satisfactorily for a corresponding use, function, or purpose. The use of any and all examples or exemplary language, as in "such as" or the like, provided herein, is intended merely to better illuminate the embodiments and does not pose a limitation on the scope of the embodiments. No language in the specification should be construed as indicating any unclaimed element as essential to the practice of the embodiments. In the description, it is understood that terms such as "first," "second," "top," "bottom," "upper," "lower," and the like are words of convenience and are not to be construed as limiting terms.

With certain details and embodiments of the present inventions for an automatic system for the conservation of oxygen and other substances disclosed, it will be appreciated by one skilled in the art that numerous changes and additions could be made thereto without deviating from the scope of the invention. This is particularly true when one bears in mind that the presently preferred embodiments merely exemplify the broader invention revealed herein. Accordingly, it will be clear that those with major features of the invention in mind could craft embodiments that incorporate those major features while not incorporating all of the features included in the preferred embodiments.

Therefore, the following claims shall define the scope of protection to be afforded to the invention. Those claims shall be deemed to include equivalent constructions insofar as they do not depart from the scope of the invention. It must be further noted that a plurality of the following claims may express, or be interpreted to express, certain elements as means for performing a specific function, at times without the recital of structure or material.

## Claims

1. A system (10) for the conservation of oxygen supplied to a patient, the system (10) comprising:
an expandable and compressible donor reservoir (404) with an outer wall, an inner volume for retaining a volume of oxygen, and at least one orifice for allowing a passage of oxygen into and out of the inner volume;
a supply conduit (408) adapted to receive oxygen from a source of oxygen (406) wherein the supply conduit (408) has a first end for supplying oxygen to the donor reservoir (404) and a second end for being fluidically connected to the source of oxygen (406); and
an ambient pressure conduit (422) adapted to supply oxygen along a fluid path from the donor reservoir (404) to a recipient wherein the ambient pressure conduit (422) has a first end in fluidic communication with the donor reservoir (404) for receiving oxygen from the donor reservoir (404) and a second end for being fluidically connected to the recipient;
**characterized in that**
the donor reservoir (404) comprises a shell of flexible foil; and
the system (10) comprises:
an inflation detection system operable to detect a first condition wherein the donor reservoir (404) is inflated with oxygen to a predetermined state of inflation and a second condition wherein the donor reservoir (404) is below the predetermined state of inflation; and
a valve system for being disposed between the source of oxygen (406) and the donor reservoir (404) wherein the valve system is operative when in a closed condition to prevent oxygen from flowing from the source of oxygen (406) and into the donor reservoir (404) when the donor reservoir (404) is in the first condition and wherein the valve system is operative in an open condition to permit oxygen to flow from the source of oxygen (406) and into the donor reservoir (404) when the donor reservoir (404) is in the second condition.

2. The system (10) for the conservation of oxygen of claim 1, **characterized in that** the valve system and the inflation detection system are operative to maintain the volume of oxygen in the donor reservoir (404) substantially at ambient pressure.

3. The system (10) for the conservation of oxygen of claim 2, **characterized in that** the donor reservoir (404) has a fully inflated condition, wherein the inflation detection system is operative to detect when the donor reservoir (404) is inflated to within a predetermined range of the fully inflated condition, wherein the inflation detection system detects the first condition when the donor reservoir (404) is inflated to within the predetermined range of the fully inflated condition, and wherein the inflation detection system detects the second condition when the donor reservoir (404) is inflated below the predetermined range of the fully inflated condition.

4. The system (10) for the conservation of oxygen of claim 1, **characterized in that** the system (10) further comprises a source of oxygen (406).

5. The system (10) for the conservation of oxygen of claim 1, **characterized in that** the inflation detection system comprises an electro mechanical system with a switch (414) disposed to be moved by the outer wall of the donor reservoir (404) when the donor reservoir (404) is inflated with oxygen to the predetermined state of inflation.

6. The system (10) for the conservation of oxygen of claim 5, **characterized in that** the switch (414) is biased toward the donor reservoir (404).

7. The system (10) for the conservation of oxygen of claim 5, **characterized in that** the switch (414) has an activated state wherein the switch (414) is disposed at or beyond an inward position with respect to the inner volume of the donor reservoir (404) and a deactivated state when the switch (414) is moved outwardly by the outer wall of the donor reservoir (404) when the volume of oxygen in the donor reservoir (404) reaches the predetermined state of inflation, wherein the valve system is operative to prevent oxygen from flowing from the source of oxygen (406) and into the donor reservoir (404) when the switch (414) is in the deactivated state and wherein the valve system is operative to permit oxygen to flow from the source of oxygen (406) and into the donor reservoir (404) when the switch is in the activated state.

8. The system (10) for the conservation of oxygen of claim 7, **characterized in that** the switch (414) comprises a float switch (414).

9. The system (10) for the conservation of oxygen of claim 8, **characterized in that** the float switch (414) comprises a contact structure (416) with a collar (440) that is extendable and retractable relative to a central column (436) and wherein the collar (440) retains a magnet (447) and wherein the central column (436) retains electrical contacts (445) that are brought into electrical contact by a proximity to the magnet (447) when the switch (414) is in the activated state.

10. The system (10) for the conservation of oxygen of claim 1, **characterized in that** the valve system comprises a solenoid valve (412) that is in electrical communication with the inflation detection system.

11. The system (10) for the conservation of oxygen of claim 10, **characterized in that** the solenoid valve (412) is induced by the inflation detection system to a closed condition to prevent the flow of oxygen from the source of oxygen to the donor reservoir (404) when the donor reservoir (404) is in the first condition and wherein the solenoid valve is induced by the inflation detection system to an open condition to permit the flow of oxygen from the source of oxygen to the donor reservoir (404) when the donor reservoir (404) is in the second condition.

12. The system (10) for the conservation of oxygen of claim 1, **characterized in that** the system (10) further comprises a recipient delivery device (426) coupled to the second end of the ambient pressure conduit.

13. The system (10) for the conservation of oxygen of claim 12, **characterized in that** the recipient delivery device (426) comprises a breathing mask (426).

14. The system (10) for the conservation of oxygen of claim 1, **characterized in that** the donor reservoir (404) is disposed within a housing (402).

15. The system (10) for the conservation of oxygen of claim 14, **characterized in that** the inflation detection system comprises an electro-mechanical system with a switch (414) supported by the housing and disposed to be moved by the outer wall of the donor reservoir (404) when the donor reservoir (404) is inflated with oxygen to the predetermined state of inflation.

16. The system (10) for the conservation of oxygen of claim 14, **characterized in that** the housing (402) is transparent whereby the state of inflation of the donor reservoir (404) can be visually perceived.

17. The system (10) for the conservation of oxygen of claim 1, **characterized in that** the system (10) further comprises a one-way inspiratory valve (424) disposed along the fluid path from the donor reservoir (404) to the recipient wherein the one-way inspiratory valve (424) is operative to enable oxygen to flow from the donor reservoir (404), through the ambient pressure conduit (422), and to the recipient but to prevent reverse flow of oxygen.

18. The system (10) for the conservation of oxygen of claim 1, **characterized in that** the inflation detection system comprises a contactless detection system (456).

19. The system (10) for the conservation of oxygen of claim 18, **characterized in that** the inflation detection system comprises an optical detection system (456).

## Patentansprüche

1. Ein System (10) zur Konservierung des einem Patienten zugeführten Sauerstoffs, wobei das System (10) Folgendes umfasst:
ein dehnbares und komprimierbares Spenderreservoir (404) mit einer Außenwand, einem Innenvolumen zur Aufnahme eines Sauerstoffvolumens und mindestens einer Öffnung zum Ein- und Austritt von Sauerstoff in das Innenvolumen;
eine Zufuhrleitung (408) zur Aufnahme von Sauerstoff aus einer Sauerstoffquelle (406), wobei die Zufuhrleitung (408) ein erstes Ende zur Sauerstoffzufuhr zum Spenderreservoir (404) und ein zweites Ende zur fluidischen Verbindung mit der Sauerstoffquelle (406) aufweist; und
eine Umgebungsdruckleitung (422) zur Sauerstoffzufuhr entlang eines Fluidpfads vom Spenderreservoir (404) zu einem Empfänger, wobei die Umgebungsdruckleitung (422) ein erstes Ende in fluidischer Verbindung mit dem Spenderreservoir (404) zur Sauerstoffaufnahme vom Spenderreservoir (404) und ein zweites Ende zur fluidischen Verbindung mit dem Empfänger aufweist;
**dadurch gekennzeichnet, dass**
das Spenderreservoir (404) eine Hülle aus einer flexiblen Folie umfasst; und
das System (10) umfasst:
ein System zur Erkennung des Aufblaszustands, das einen ersten Zustand erkennt, in dem das Spenderreservoir (404) mit Sauerstoff bis zu einem vorbestimmten Aufblaszustand aufgeblasen ist, und einen zweiten Zustand, in dem sich das Spenderreservoir (404) unterhalb des vorbestimmten Aufblaszustands befindet; und
ein Ventilsystem, das zwischen der Sauerstoffquelle (406) und dem Spenderreservoir (404) angeordnet ist, wobei das Ventilsystem im geschlossenen Zustand den Sauerstofffluss von der Sauerstoffquelle (406) in das Spenderreservoir (404) verhindert, wenn sich das Spenderreservoir (404) im ersten Zustand befindet, und im geöffneten Zustand den Sauerstofffluss von der Sauerstoffquelle (406) in das Spenderreservoir (404) ermöglicht, wenn sich das Spenderreservoir (404) im zweiten Zustand befindet.

2. Das System (10) zur Konservierung von Sauerstoff gemäß Anspruch 1, **dadurch gekennzeichnet, dass** das Ventilsystem und das System zur Erkennung des Aufblaszustands so arbeiten, dass das Sauerstoffvolumen im Spenderreservoir (404) im Wesentlichen auf Umgebungsdruck gehalten wird.

3. Das System (10) zur Konservierung von Sauerstoff gemäß Anspruch 2, **dadurch gekennzeichnet, dass** sich das Spenderreservoir (404) in einem vollständig aufgeblasenen Zustand befindet, wobei das System zur Erkennung des Aufblaszustands so funktioniert, dass es erkennt, wann das Spenderreservoir (404) innerhalb eines vorbestimmten Bereichs des vollständig aufgeblasenen Zustands aufgeblasen ist, wobei das System zur Erkennung des Aufblaszustands den ersten Zustand erkennt, wenn das Spenderreservoir (404) innerhalb des vorbestimmten Bereichs des vollständig aufgeblasenen Zustands aufgeblasen ist, und wobei das System zur Erkennung des Aufblaszustands den zweiten Zustand erkennt, wenn das Spenderreservoir (404) unterhalb des vorbestimmten Bereichs des vollständig aufgeblasenen Zustands aufgeblasen ist.

4. Das System (10) zur Konservierung von Sauerstoff gemäß Anspruch 1, **dadurch gekennzeichnet, dass** das System (10) ferner eine Sauerstoffquelle (406) umfasst.

5. Das System (10) zur Konservierung von Sauerstoff gemäß Anspruch 1, **dadurch gekennzeichnet, dass** das System zur Erkennung des Aufblaszustands ein elektromechanisches System mit einem Schalter (414) umfasst, der von der Außenwand des Spenderreservoirs (404) bewegt wird, wenn das Spenderreservoir (404) mit Sauerstoff auf den vorbestimmten Aufblaszustand aufgeblasen wird.

6. Das System (10) zur Konservierung von Sauerstoff gemäß Anspruch 5, **dadurch gekennzeichnet, dass** der Schalter (414) in Richtung des Spenderreservoirs (404) vorgespannt ist.

7. Das System (10) zur Konservierung von Sauerstoff gemäß Anspruch 5, **dadurch gekennzeichnet, dass** der Schalter (414) einen aktivierten Zustand aufweist, in dem sich der Schalter (414) in Bezug auf das Innenvolumen des Spenderreservoirs (404) in oder jenseits einer nach innen gerichteten Position befindet, und einen deaktivierten Zustand, in dem der Schalter (414) durch die Außenwand des Spenderreservoirs (404) nach außen bewegt wird, wenn das Sauerstoffvolumen im Spenderreservoir (404) den vorbestimmten Füllstand erreicht hat, wobei das Ventilsystem so arbeitet, dass Sauerstoff nicht von der Sauerstoffquelle (406) in das Spenderreservoir (404) strömen kann, wenn sich der Schalter (414) im deaktivierten Zustand befindet, und wobei das Ventilsystem so arbeitet, dass Sauerstoff von der Sauerstoffquelle (406) in das Spenderreservoir (404) strömen kann, wenn sich der Schalter im aktivierten Zustand befindet.

8. Das System (10) zur Konservierung von Sauerstoff gemäß Anspruch 7, **dadurch gekennzeichnet, dass** der Schalter (414) einen Schwimmerschalter (414) umfasst.

9. Das System (10) zur Konservierung von Sauerstoff gemäß Anspruch 8, **dadurch gekennzeichnet, dass** der Schwimmerschalter (414) eine Kontaktstruktur (416) mit einem Kragen (440) umfasst, der relativ zu einer zentralen Säule (436) ausziehbar und einziehbar ist, wobei der Kragen (440) einen Magneten (447) hält und wobei die zentrale Säule (436) elektrische Kontakte (445) hält, die durch die Nähe zum Magneten (447) in elektrischen Kontakt gebracht werden, wenn sich der Schalter (414) im aktivierten Zustand befindet.

10. Das System (10) zur Konservierung von Sauerstoff gemäß Anspruch 1, **dadurch gekennzeichnet, dass** das Ventilsystem ein Magnetventil (412) umfasst, das in elektrischer Verbindung mit dem System zur Erkennung des Aufblaszustands steht.

11. Das System (10) zur Konservierung von Sauerstoff gemäß Anspruch 10, **dadurch gekennzeichnet, dass** das Magnetventil (412) durch das System zur Erkennung des Aufblaszustands in einen geschlossenen Zustand versetzt wird, um den Sauerstofffluss von der Sauerstoffquelle zum Spenderreservoir (404) zu verhindern, wenn sich das Spenderreservoir (404) im ersten Zustand befindet, und wobei das Magnetventil durch das System zur Erkennung des Aufblaszustands in einen offenen Zustand versetzt wird, um den Sauerstofffluss von der Sauerstoffquelle zum Spenderreservoir (404) zu ermöglichen, wenn sich das Spenderreservoir (404) im zweiten Zustand befindet.

12. Das System (10) zur Konservierung von Sauerstoff gemäß Anspruch 1, **dadurch gekennzeichnet, dass** das System (10) ferner eine Empfängerabgabevorrichtung (426) umfasst, die mit dem zweiten Ende der Umgebungsdruckleitung verbunden ist.

13. Das System (10) zur Konservierung von Sauerstoff gemäß Anspruch 12, **dadurch gekennzeichnet, dass** die Empfängerabgabevorrichtung (426) eine Atemmaske (426) umfasst.

14. Das System (10) zur Konservierung von Sauerstoff gemäß Anspruch 1, **dadurch gekennzeichnet, dass** das Spenderreservoir (404) in einem Gehäuse (402) angeordnet ist.

15. Das System (10) zur Konservierung von Sauerstoff gemäß Anspruch 14, **dadurch gekennzeichnet, dass** das System zur Erkennung des Aufblaszustands ein elektromechanisches System mit einem Schalter (414) umfasst, der durch das Gehäuse getragen wird und so ausgelegt ist, dass er durch die Außenwand des Spenderreservoirs (404) bewegt wird, wenn das Spenderreservoir (404) mit Sauerstoff auf den vorbestimmten Aufblaszustand aufgeblasen wird.

16. Das System (10) zur Konservierung von Sauerstoff gemäß Anspruch 14, **dadurch gekennzeichnet, dass** das Gehäuse (402) transparent ist, sodass der Aufblaszustand des Spenderreservoirs (404) visuell erkennbar ist.

17. Das System (10) zur Konservierung von Sauerstoff gemäß Anspruch 1, **dadurch gekennzeichnet, dass** das System (10) ferner ein Einweg-Einatemventil (424) umfasst, das entlang des Fluidwegs vom Spenderreservoir (404) zum Empfänger angeordnet ist, wobei das Einweg-Einatemventil (424) so wirkt, dass Sauerstoff vom Spenderreservoir (404) durch die Umgebungsdruckleitung (422) und zum Empfänger fließen kann, ein Rückfluss von Sauerstoff jedoch verhindert wird.

18. Das System (10) zur Konservierung von Sauerstoff gemäß Anspruch 1, **dadurch gekennzeichnet, dass** das System zur Erkennung des Aufblaszustands ein berührungsloses Erkennungssystem (456) umfasst.

19. Das System (10) zur Konservierung von Sauerstoff gemäß Anspruch 18, **dadurch gekennzeichnet, dass** das System zur Erkennung des Aufblaszustands ein optisches Detektionssystem (456) umfasst.

## Revendications

1. Un système (10) pour la conservation de l'oxygène fourni à un patient,
le système (10) comprenant :
un réservoir donneur (404) expansible et compressible, ayant une paroi extérieure, un volume intérieur pour retenir un volume d'oxygène, et au moins un orifice pour permettre le passage de l'oxygène vers et depuis le volume intérieur,
une conduite d'alimentation (408), adaptée pour recevoir de l'oxygène provenant d'une source d'oxygène (406), la conduite d'alimentation (408) ayant une première extrémité pour fournir de l'oxygène au réservoir donneur (404) et une deuxième extrémité pour être relié de manière fluidique à la source d'oxygène (406) ; et
un conduit à pression ambiante (422), adapté pour fournir de l'oxygène le long d'un chemin fluidique depuis le réservoir donneur (404) vers un récepteur, le conduit à pression ambiante (422) présentant une première extrémité en communication fluidique avec le réservoir donneur (404) pour recevoir de l'oxygène provenant du réservoir donneur (404) et une deuxième extrémité destinée à être reliée de manière fluidique au récepteur ;
**caractérisé en ce que**
le réservoir donneur (404) comprend une enveloppe en feuille flexible ; et
le système (10) comprend :
un système de détection de gonflage, conçu de manière à détecter une première condition dans laquelle le réservoir donneur (404) est gonflé avec de l'oxygène jusqu'à un état de gonflage prédéterminé et une deuxième condition dans laquelle le réservoir donneur (404) est en dessous de l'état de gonflage prédéterminé ; et
un système de valve, destiné à être agencé entre la source d'oxygène (406) et le réservoir donneur (404), le système de valve étant conçu de manière à, lorsqu'il est dans un état fermé, empêcher l'oxygène de s'écouler de la source d'oxygène (406) vers le réservoir donneur (404) lorsque le réservoir donneur (404) se trouve dans la première condition, et le système de valve étant conçu de manière à, dans une condition ouverte, permettre à l'oxygène de s'écouler depuis la source d'oxygène (406) vers le réservoir donneur (404) lorsque le réservoir donneur (404) se trouve dans la deuxième condition.

2. Le système (10) pour la conservation de l'oxygène selon la revendication 1, **caractérisé en ce que** le système de valve et le système de détection de gonflage sont conçus de manière à maintenir le volume d'oxygène dans le réservoir donneur (404) sensiblement à la pression ambiante.

3. Le système (10) pour la conservation de l'oxygène selon la revendication 2, **caractérisé en ce que** le réservoir donneur (404) a un état complètement gonflé, le système de détection de gonflage étant conçu de manière à détecter lorsque le réservoir donneur (404) est gonflé jusqu'à une plage prédéterminée de l'état complètement gonflé, le système de détection de gonflage détectant le premier état lorsque le réservoir donneur (404) est gonflé dans la plage prédéterminée de l'état de gonflage complet, et le système de détection de gonflage détectant le deuxième état lorsque le réservoir donneur (404) est gonflé en dessous de la plage prédéterminée de l'état de gonflage complet.

4. Le système (10) pour la conservation de l'oxygène selon la revendication 1, **caractérisé en ce que** le système (10) comprend en outre une source d'oxygène (406).

5. Le système (10) pour la conservation de l'oxygène selon la revendication 1, **caractérisé en ce que** le système de détection de gonflage comprend un système électromécanique avec un commutateur (414) agencé de manière à être déplacé par la paroi extérieure du réservoir donneur (404) lorsque le réservoir donneur (404) est gonflé avec de l'oxygène jusqu'à l'état de gonflage prédéterminé.

6. Le système (10) pour la conservation de l'oxygène selon la revendication 5, **caractérisé en ce que** le commutateur (414) est sollicité vers le réservoir donneur (404).

7. Le système (10) pour la conservation de l'oxygène selon la revendication 5, **caractérisé en ce que** le commutateur (414) a un état activé dans lequel le commutateur (414) est agencé à une position intérieure ou au-delà par rapport au volume intérieur du réservoir donneur (404) et un état désactivé lorsque le commutateur (414) est déplacé vers l'extérieur par la paroi extérieure du réservoir donneur (404) lorsque le volume d'oxygène dans le réservoir donneur (404) atteint l'état de gonflage prédéterminé, le système de valve étant conçu de manière à empêcher l'oxygène de s'écouler de la source d'oxygène (406) vers le réservoir donneur (404) lorsque le commutateur (414) est dans l'état désactivé, et le système de valve étant conçu de manière à permettre à l'oxygène de s'écouler depuis la source d'oxygène (406) et dans le réservoir donneur (404) lorsque le commutateur est dans l'état activé.

8. Le système (10) pour la conservation de l'oxygène selon la revendication 7, **caractérisé en ce que** le commutateur (414) comprend un commutateur à flotteur (414).

9. Le système (10) pour la conservation de l'oxygène selon la revendication 8, **caractérisé en ce que** l'interrupteur à flotteur (414) comprend une structure de contact (416) avec un collier (440) qui est apte à être déployé et rétracté par rapport à une colonne centrale (436), et dans lequel le collier (440) retient un aimant (447), et dans lequel la colonne centrale (436) retient des contacts électriques (445) qui sont mis en contact électrique par proximité avec l'aimant (447) lorsque le commutateur (414) est à l'état activé.

10. Le système (10) pour la conservation de l'oxygène selon la revendication 1, **caractérisé en ce que** le système de valve comprend une électrovalve (412) qui est en communication électrique avec le système de détection de gonflage.

11. Le système (10) pour la conservation de l'oxygène selon la revendication 10, **caractérisé en ce que** l'électrovalve (412) est induite par le système de détection de gonflage dans un état fermé pour empêcher l'écoulement d'oxygène de la source d'oxygène vers le réservoir donneur (404) lorsque le réservoir donneur (404) est dans la première condition et la valve solénoïde étant induite par le système de détection de gonflage dans une condition ouverte pour permettre l'écoulement d'oxygène depuis la source d'oxygène vers le réservoir donneur (404) lorsque le réservoir donneur (404) est dans la deuxième condition.

12. Le système (10) pour la conservation de l'oxygène selon la revendication 1, **caractérisé en ce que** le système (10) comprend en outre un dispositif (426) d'administration au receveur relié à la deuxième extrémité du conduit à pression ambiante.

13. Le système (10) pour la conservation de l'oxygène selon la revendication 12, **caractérisé en ce que** le dispositif (426) d'administration au receveur comprend un masque respiratoire (426).

14. Le système (10) pour la conservation de l'oxygène selon la revendication 1, **caractérisé en ce que** le réservoir donneur (404) est agencé à l'intérieur d'un boîtier (402).

15. Le système (10) pour la conservation de l'oxygène selon la revendication 14, **caractérisé en ce que** le système de détection de gonflage comprend un système électromécanique avec un interrupteur (414) supporté par le boîtier et agencé de manière à être déplacé par la paroi extérieure du réservoir donneur (404) lorsque le réservoir donneur (404) est gonflé avec de l'oxygène jusqu'à l'état de gonflage prédéterminé.

16. Le système (10) pour la conservation de l'oxygène selon la revendication 14, **caractérisé en ce que** le boîtier (402) est transparent, ce qui permet de percevoir visuellement l'état de gonflage du réservoir donneur (404).

17. Le système (10) pour la conservation de l'oxygène selon la revendication 1, **caractérisé en ce que** le système (10) comprend en outre une valve inspiratoire unidirectionnelle (424) agencée le long du trajet du fluide entre le réservoir donneur (404) et le receveur, la valve inspiratoire unidirectionnelle (424) étant conçue de manière à permettre à l'oxygène de s'écouler du réservoir donneur (404) à travers le conduit à pression ambiante (422) et vers le récepteur, mais empêche le reflux de l'oxygène.

18. Le système (10) pour la conservation de l'oxygène selon la revendication 1, **caractérisé en ce que** le système de détection de gonflage comprend un système de détection sans contact (456).

19. Le système (10) pour la conservation de l'oxygène selon la revendication 18, **caractérisé en ce que** le système de détection de gonflage comprend un système de détection optique (456).
